# EUROPEAN PATENT SPECIFICATION

(11) **EP 0 921 765 B1**
(45) Date of publication and mention of the grant of the patent: **02.05.2007**
(21) Application number: 97908960.4
(22) Date of filing: 04.03.1997
(51) Int. Cl.: A61B 18/12

(54) **VASCULAR CATHETER-BASED SYSTEM FOR HEATING TISSUE**
VASKULARES KATHETERSYSTEM ZUM ERWÄRMEN VON GEWEBEN
SYSTEME UTILISANT UN CATHETER VASCULAIRE POUR RECHAUFFER DES TISSUS

(30) Priority: 05.03.1996 US 610911; 26.09.1996 US 717994; 26.09.1996 US 720209
(43) Date of publication of application: 16.06.1999
(73) Proprietor: VNUS MEDICAL TECHNOLOGIES, INC., Sunnyvale, CA 94086 (US)
(72) Inventor: LAUFER, Michael, D., Menlo Park, CA 94025 (US); FARLEY, Brian, E., Los Altos, CA 94024 (US); SCHULZ, Grace, Y., San Carlos, CA 94070 (US); ZIKORUS, Arthur, W., San Jose, CA 95131 (US); PARKER, Mark, P., San Jose, CA 95118 (US)
(74) Representative: McLeish, Nicholas Alistair Maxwell
(86) International application number: PCT/US1997/003637
(87) International publication number: WO 1997/032532

(56) References cited:
- EP-A- 0 189 329
- WO-A-92/12681
- WO-A-94/07446
- WO-A-95/10322
- DE-A- 3 516 830

## Description

### BACKGROUND OF THE INVENTION

This invention relates to a catheter-based system to position an electrode for providing energy to shrink a vein intraluminally to change the fluid flow dynamics and to restore the competency of venous valves and the proper function of the vein in a minimally invasive procedure.

The human venous system of the lower limb consists essentially of the superficial venous system and the deep venous system with perforating veins connecting the two systems. The superficial system includes the long or great saphenous vein and the short saphenous vein. The deep venous system includes the anterior and posterior tibial veins which unite to form the popliteal vein, which in turn becomes the femoral vein when joined by the short saphenous vein.

The venous systems contain numerous one-way valves for directing blood flow back to the heart. Venous valves are usually bicuspid valves, with each cusp forming a sack or reservoir for blood which, under pressure, forces the free surfaces of the cusps together to prevent retrograde flow of the blood and allow antegrade flow to the heart. When an incompetent valve is in the flow path of retrograde flow toward the foot, the valve is unable to close because the cusps do not form a proper seal and retrograde flow of blood cannot be stopped.

Incompetent valves in the venous system can occur with vein dilation. Separation of the cusps of the venous valve at the commissure may occur as a result. The leaflets are stretched by the dilation of the vein and concomitant increase in the vein diameter which the leaflets traverse. Stretching of the leaflets of the venous valve allows the loose leaflets to fold on themselves and leave the valve open. This prolapse can allow reflux of blood in the vein. Eventually the venous valve fails, thereby increasing the pressure on the lower venous sections and overlying tissues. Two venous diseases which often involve vein dilation are varicose veins and chronic venous insufficiency.

The varicose vein condition includes dilation and tortuosity of the superficial veins of the lower limb, resulting in unsightly discoloration, pain and ulceration. Varicose veins often involve incompetence of one or more venous valves, which allow reflux of blood from the deep venous system to the superficial venous system or reflux within the superficial system. Current treatments include such invasive open surgical procedures as vein stripping, sclerotherapy, and occasionally, vein grafting, venous valvuloplasty, and the implantation of various prosthetic devices. The removal of varicose veins from the body can be a tedious, time-consuming procedure having a painful and slow healing process. Complications, scarring, and the loss of the vein for future cardiac and other by-pass procedures may also result. Along with the complications and risks of invasive open surgery, varicose veins may persist or reoccur, particularly when the valvular problem is not corrected. Due to the long, arduous, and tedious nature of the surgical procedure, treating multiple venous sections can exceed the physical stamina of the physician, and thus render complete treatment of the varicose vein conditions impractical.

Chronic venous insufficiency (CVI) is a problem caused by hydrodynamic forces acting on the tissues of the body, especially the legs, ankles and feet. As the veins dilate due to increased pressure, the valves in the veins fail. This causes the pressure to increase on the next valve and vein segment down, causing those veins to dilate, and as this continues, the valves in the veins eventually all fail. As they fail, the effective height of the column of blood above the feet and ankles grows, and the weight and hydrostatic pressure exerted on the tissues of the ankle and foot increases. When the weight of that column reaches a critical point from the valve failures, ulcerations of the ankle begin to form, which start deep and eventually come to the surface. These ulcerations do not heal easily because the weight of blood which caused them continues to persist, and have the tendency to enlarge the ulcer.

Chronic venous insufficiency often consists of hypertension of the lower limb in the deep, perforating and often superficial veins, and may result in discoloration, pain, swelling and ulceration. Existing treatments for chronic venous insufficiency are often less than ideal. These treatments include the elevation of the legs, compressing the veins externally with elastic support hose, and surgical repair by grafting vein sections with healthy valves from the arm into the leg. These methods have variable effectiveness. Moreover, invasive surgery has its associated complications with risk to life and expense. Similarly, the palliative therapies require major lifestyle changes for the patient. For example, the ulcers will reoccur unless the patient continues to elevate the legs and use support hose continuously throughout the life of the patient.

Due to the time-consuming and invasive nature of the current surgical treatments, such as vein grafting, typically only one valve is treated during any single procedure. This greatly limits the ability of the physician to fully treat patients suffering from chronic venous insufficiency. Every instance of invasive surgery, however, has its associated complications with risk to life and expense.

The ligation of vascular lumen by tying a suture around them, cauterization or coagulation using electrical energy from an electrode has been employed as an alternative to stripping, or the surgical removal of such veins. However, ligation procedures close off the lumen, essentially destroying their functional capability. For example, it is known to introduce an electrode into the leg of a patient, and position the electrode adjacent to the exterior of the varicose veins to be treated. Through a small stab incision, a probe is forced through the subcutaneous layer between the fascia and the skin, and then to the various veins to be destroyed. Electrodes at the outer end of the probe are placed adjacent to the varicose veins. Once properly positioned, an alternating current of 500 kilohertz is applied to destroy the adjacent varicose veins. The veins lose the function of allowing blood to flow through, and are no longer of use. For example, ligating the saphenous vein would render that vein unavailable for harvesting in other surgical procedures such as coronary by-pass operations. Ligation techniques which functionally destroy the vein lumen would appear be inappropriate to a corrective procedure for restoring and maintaining the function of the vein.

Hemorrhoids are dilated veins in and around the anus and lower rectum. Dilation may result from an increased pressure in the hemorrhoidal vein. Constipation, including the frequent straining to pass hard stools increases pressure in hemorrhoidal veins, is a common cause of hemorrhoids. Other contributing factors include pregnancy, a low fiber diet, and obesity. As the hemorrhoidal vein becomes more dilated from the increased pressure, the venous valves of the hemorrhoidal vein may begin to fail and become incompetent. This can exacerbate the dilation of the hemorrhoidal vein as reflux of blood is allowed in the vein by the open incompetent valve. The vein may eventually form a sac-like protrusion if the condition is allowed to persist. Hemorrhoids are generally classified as being either internal or external, depending on their location relative to the dentate line. The dentate line is easily identified as the demarcation between the pink mucosa that form the anoderm. The dentate line separates the internal and external hemorrhoid systems. Internal hemorrhoids are located inside the anus above the dentate line. External hemorrhoids are located below the dentate line. Either can extend out of the anus.

Straining or irritation caused by passing stool can injure the delicate surface of an internal hemorrhoid and cause bleeding. If the pressure and dilation of the hemorrhoidal vein continues, the internal hemorrhoids may prolapse and be forced through the anal opening. If a hemorrhoid remains prolapsed, considerable discomfort, including itching and bleeding, may result. The blood supply to these prolapsed hemorrhoids may become cut off by the anal sphincter, which gives rise to a strangulated hemorrhoid. Thrombosis may result where the blood within the prolapsed vein becomes clotted. This extremely painful condition can cause edema and inflammation.

Increased pressure in the portal venous system can also cause an increase in pressure of the superior hemorrhoidal vein (SHV) leading to an increased diameter of the hemorrhoid. The portal venous system allows venous drainage from the intestinal tissues to the liver, and can become hypertensive when the lever is cirrhotic.

The treatment methods for hemorrhoids include invasive surgery to remove the hemorrhoid, elastic ring ligation, sclerotherapy, and the application of topical ointments or suppositories. The surgical removal of extensive or severe hemorrhoids is known as a hemorrhoidectomy. This surgical procedure can be used on both internal and external hemorrhoids. However, such surgery typically involves a long recovery period, along with the associated risks and expense of invasive surgery.

Internal hemorrhoids may be treated by rubber band ligation, where a legator is inserted through a scope in the anal canal. The hemorrhoid is grasped with forceps in the legator and held in position. The legator includes a cylinder which is slid upwards and releases one or more rubber bands around the base of the hemorrhoid. A typical diameter for the rubber band is one millimeter. The band cuts off the circulation of blood to the hemorrhoid, and the hemorrhoid begins to wither away. Provided the rubber band remains in place, the hemorrhoid typically drops off within seven to ten days.

Sclerotherapy, another treatment for hemorrhoids, involves injecting a solution, such as sodium morrhuate or phenol oil, submucously into the areolar tissue around the hemorrhoidal vein to cause inflammation and scarring to eliminate the hemorrhoid. Other external treatments cause burning or coagulation to destroy the hemorrhoid. In infrared coagulation, infrared light may be applied to create a small tissue-destroying burn around the base of the hemorrhoid to cut off the blood supply to the hemorrhoid. Electrocoagulation, sometimes referred to as bipolar diathermy, may be utilized in a similar manner. In laser therapy, also known as vaporization, a laser beam causes a superficial burn to seal off the blood vessels and retain the hemorrhoid in a non-prolapsed position.

The prior treatments for hemorrhoids involving external ligation or excision of the hemorrhoid may not affect the underlying causes which gave rise to the hemorrhoidal condition initially. Thus the condition may recur.

Varicose veins called esophageal varices can form in the venous system along the submucosa of the lower esophagus, and bleeding can occur from the dilated veins. Blood returns to the heart from the portal venous system through the veins surrounding the esophagus. Unlike other veins, such as the saphenous vein in the lower leg, the veins surrounding the esophagus typically do not have valves for bringing blood back to the heart. The venous pressure in these esophageal veins is relatively high, and blood can flow back to the heart without aid of venous valves.

Esophageal varices may result from portal hypertension and other abnormalities in the portal venous system, such as cirrhosis of the liver. Bleeding or hemorrhaging may result from esophageal varices, which can be difficult to stop and, if untreated, could develop into a life threatening condition. Such varices can erode easily, and lead to a massive gastrointestinal hemorrhage.

Treatments for esophageal varices include portal-caval shunts, endoscope variceal ligation, sclerotherapy, and electrocoagulation from an electrode within the esophagus, such as from a tamponade device. The portal shunt involves the surgical joining of two veins, the portal vein and the inferior vena cava, to relieve pressure in the vein carrying blood into the liver. Although effective in eliminating recurrent hemorrhaging from varices, the attendant risks and complications of such invasive surgery, including encephalopathy and post-shunt hepatic failure, still exist for the portal shunt operation.

Endoscopic variceal ligation is analogous to rubber band ligation for treating hemorrhoids. The esophageal varices are ensnared with elastic bands to eradicate the varices. An endoscope is introduced into the patient and is placed adjacent to the esophageal varices to be treated. The varix is drawn into a drum attached to the tip of the endoscope. An elastic band mounted on the drum is then released over the varix. Endoscope variceal ligation may not achieve complete fibrosis of the inner wall of the esophagus, and recurrence of the varices may result. Other complications include bleeding from ulcers induced by the elastic bands, and esophageal obstruction due to occlusion of the lumen by banded esophageal varices.

In sclerotherapy, a solution, such as sodium morrhuate or ethanolamine, is injected submucosally into the tissue around the varicose vein in the esophagus to cause inflammation and scarring to close off the vein and reduce the likelihood of bleeding. Sclerotherapy, however, may create ulcerations which can lead to esophageal strictures.

Electrocoagulation has also been used to treat esophageal varices. A tamponade device having a metalized surface is introduced into the esophagus. The metalized surface is brought into contact with the mucous membrane of the esophagus. An electric current is then applied to the metalized surface to cause a thrombosing of the esophageal varices. This procedure may be employed to stop immediate hemorrhaging of the esophageal varices.

EP 0,205,851 discloses a catheter comprising at least one lumen, closed at the front end, for the local treatment of internal body structures, in particular stenoses. The catheter comprises at least one opening in the catheter wall which is arranged in a treatment section at the front end and which extends from the lumen to the outer wall of the catheter. The catheter also comprises an extendable and expandable part arranged in the treatment section of the catheter end with at least one electrically conductive zone which forms part of the extension of this part. The electrically conductive zone being arranged on the outer surface of this part of the catheter.

The prior treatments for esophageal varices typically involve external coagulation or obliteration of the veins, and often require multiple treatment sessions. Such treatments do not treat the varicosity directly, and may not affect the underlying causes which gave rise to the esophageal varices initially.

A need exists in the art for a system to treat dilated veins, such as those resulting in varicose veins or from venous insufficiency, which maintains the patency of the veins for venous function and yet restores valvular competency. A need also exists in the art to treat dilated hemorrhoidal veins to reduce venous pressure on the hemorrhoidal region. Such treatment should maintain the functional patency of the vein and restore valvular competency at the origins of the hemorrhoids as well as within the hemorrhoid itself. A need exists in the art to treat the dilated veins which give rise to esophageal varices and reduce venous pressure on the esophageal region from the portal vein system without the attendant risks of invasive surgery. Further need exists to provide a less invasive procedure which can treat multiple venous sites quickly and easily. The need exists to restore and normalize flow patterns, dynamics, and pressure, and shrink sections of dilated veins to a normal or reduced diameter. Where bleeding occurs, there is a need to achieve hemostasis in bleeding varices and minimize recurrence of bleeding.

### SUMMARY OF THE INVENTION

Briefly, and in general terms, the use of present invention provides a less invasive and faster method for solving the underlying problems of varicose veins and venous insufficiency, and uses a novel repair system, including a catheter for placement of an electrode for delivering radio frequency energy.

According to the present invention there is provided an apparatus for applying energy to reduce the diameter of a vein as claimed in claim 1.

The method of using the apparatus of the present invention includes the steps of introducing a catheter having a working end and means for heating located at the working end, to a treatment site in a vein; positioning the means for heating at the treatment site in the vein; applying energy from the means for heating to controllably heat the treatment site and cause shrinkage of the vein; and terminating the emission of energy from the means for heating after sufficient shrinkage of the vein has occurred so as to restore valvular competency or so that the vein remains patent so as to continue to function as a blood conduit. The method is a minimally invasive procedure which eliminates the need for open surgical procedures for venous repair, including venous valvuloplasty, and the transplantation of an arm vein into the leg.

An apparatus applies radiant energy to cause shrinkage of a vein. The heating device may include RF electrodes to heat and shrink the vein. Feedback control systems may be applied to control the application of energy to heat the venous tissue, in order to control the amount of shrinkage.

Features of the present invention include restoring the competence of venous valves, normalizing flow patterns, dynamics, and pressure, and reducing sections of dilated varicose veins to a normal diameter for cosmetic purposes. The treated veins remain patent and can continue to function and return blood to the heart.

One procedure is for restoring venous valvular competency by controllably shrinking the otherwise dilated lumen of the vein to a desired diameter.

Another advantage is to control or adjust the effective diameter of the catheter or electrode configuration in order to control the amount of circumferential shrinking experienced by the vein wall. An extendable member located adjacent to the working end of the catheter can increase the effective diameter of the catheter and limit the shrinkage of the vein.

Advantageously a catheter electrode is provided which generates a radio frequency field around the circumference of the catheter in order to shrink the vein wall circumferentially and omnidirectionally while minimizing lengthwise contraction when the catheter electrode is positioned intraluminally within the vein

A field may be generated at a specific frequency around the catheter in order to minimize coagulation within the vein, and to control the spread of heating within the venous tissue.

The venous valve leaflets are protected by minimizing the heating effect on the venous valves by the selective positioning of the electrodes within the vein.

Preferably cooling fluid is delivered to the bloodstream in order reduce the likelihood of heating the blood to a point of coagulation.

Preferably shrinkage of the vein past the end of the catheter is prevented.

Advantageously the electrodes are maintained in apposition to the venous tissue to ensure that the heat is delivered towards the venous tissue, and not the blood moving through the vein.

The bowable members are deflected radially outward for maintaining contact with the venous tissue. The bowable members are conductive longitudinal electrodes substantially covered by an insulating film, except for the portion which is to come into apposition with the venous tissue.

In another arrangement a balloon located on one side of the catheter having electrodes on the opposite side. Inflation of the balloon will move the electrodes into apposition with the vein wall on the opposite side.

An advantage of the use of the apparatus according to the present invention is to provide a procedure which can treat multiple venous sites quickly and easily.

An advantage of the present invention is that no foreign object or prosthesis remain in the vasculature after treatment.

In the method of using the apparatus according to the present invention, advantageously the step of positioning the means for heating at the treatment site further includes the step of placing the means for heating adjacent the venous valves at the treatment site to restore venous valve competency.

Advantageously the step of positioning the means for heating further includes the step of arranging the means for heating for achieving circumferential shrinkage of the vein and minimizing axial shortening.

Advantageously the step of positioning the means for heating further includes the step of moving the means for heating into apposition with the vein wall at the treatment site.

Advantageously the step of positioning further includes the step of increasing an effective diameter of the catheter to place the means for heating into apposition with the vein wall; and the step of applying energy further includes the step of reducing the effective diameter of the catheter in a controlled manner so as to maintain apposition with the vein wall as the vein wall shrinks, until a diameter for the vein is achieved for restoring venous function.

Advantageously the method further comprises the step of limiting the shrinkage of the vein to a diameter defined by the heating means. Preferably, the method further comprises the step of moving the heating means radially outward from the catheter with a bowable member so as to place the heating means in apposition with the vein. Preferably the step of applying energy further includes the step of controlling the energy from the means for heating so as to control the depth of heating at the treatment site of the vein.

Advantageously the method further comprises the step of determining the extent of shrinkage of the vein.

Advantageously the method further comprises the step of determining the extent of shrinkage of the vein using fluoroscopy.

Advantageously the method further comprises the step of determining the extent of shrinkage of the vein using ultrasound.

These and other aspects and advantages of the present invention will become apparent from the following more detailed description, when taken in conjunction with the accompanying drawings which illustrate, by way of example, the principles of the invention.

### BRIEF DESCRIPTION OF THE DRAWINGS

FIGURE 1 shows a cross-sectional view of a dilated vein having incompetent venous valves in a lower limb which are to be treated by using the present invention;
FIGURE 2 shows a representative view of a venous section from FIG. 1 taken along lines 2-2 which is to be treated by using the present invention;
FIGURE 3 shows a partial cross-sectional view of a catheter having electrodes being delivered antegrade to a venous treatment site.
FIGURE 4 shows the partial cross-sectional view of the venous section of FIG. 2 after being treated by using the present invention;
FIGURE 5 shows a partial cross-sectional view of the catheter and vein shown in FIGURE 3 being delivered to another venous treatment site.
FIGURE 6 shows a partial cross-sectional view of a catheter being delivered retrograde and deflected laterally to a venous treatment site;
FIGURE 7 shows a partial cross-sectional view of an arrangement of the catheter having a bulbous up and ring electrodes for treating a dilated vein in accordance with the present invention;
FIGURE 8 shows a partial cross-sectional view of an arrangement of the catheter having a flush up at the working end and ring electrodes for treating a dilated vein;
FIGURE 9 shows a partial cross-sectional view of an arrangement of the catheter having a cap electrode for treating a dilated vein;
FIGURE 10 shows a partial cross-sectional view of another arrangement of the catheter having a cap electrode and a balloon to center the placement of the electrode within the vein to be treated;
FIGURES 11a, 11b, and 11c show partial cross-sectional views of another arrangement of the catheter having a bendable tip which deflects laterally for causing apposition between the electrodes of the catheter and the vein wall;
FIGURES 12a and 12b show partial cross-sectional side and top views, respectively, of another arrangement of the catheter having a balloon on one side of the catheter and longitudinal electrodes on the other side at the working end of the catheter for moving the electrodes into appositional contact with the vein wall;
FIGURE 13 shows an embodiment of the catheter having bendable electrodes which deflect outwardly for increasing the effective diameter at the working end of the catheter in accordance with the invention;
FIGURE 14 shows another arrangement of the catheter having a balloon and bendable members with electrodes which deflect outwardly for increasing the effective diameter at the working end of the catheter;
FIGURE 15a shows a cross-sectional view of an arrangement of the catheter shown in FIGURE 14 having four equidistantly spaced electrodes;
FIGURE 15b shows a cross-sectional view of an arrangement of the catheter shown in FIGURE 14 having four electrodes preferentially spaced to form two pairs of electrodes;
FIGURE 16 shows a partial cross-sectional view of another arrangement of the catheter having four equidistantly spaced electrodes, and being delivered retrograde to a venous treatment site;
FIGURE 17 shows a partial cross-sectional view of an arrangement of an over-the-wire balloon catheter having four equidistantly spaced apart electrodes on the surface of the balloon;
FIGURE 18 shows a cross-sectional view taken along the lines 18-18 of the over-the-wire balloon catheter of FIG. 17;
FIGURE 19 shows a partial cross-sectional view of another arrangement of the catheter having electrodes located within the balloon portion;
FIG. 20 is a side view of an embodiment of a catheter having bowable electrodes in accordance with the invention coupled with a block diagram of a heat treatment system;
FIG. 21 is a partial side view of the working end of the catheter illustrated in FIG. 20, and having electrodes which deflect outwardly for increasing the effective diameter at the working end of the catheter in accordance with the present invention;
FIG. 22 is a cross-sectional view along lines 22-22 of the electrode for the catheter depicted in FIG. 21;
FIG. 23 is a cross-sectional view along lines 23-23 of FIG. 20, and depicts a catheter having four equidistantly spaced electrodes in accordance with the present invention;
FIG. 24 is a cross-sectional view of another embodiment of the catheter depicted in FIG. 23, this embodiment having four electrodes preferentially spaced to form two pairs of electrodes in accordance with the present invention;
FIG. 25 is a cross-sectional view of another embodiment of the catheter depicted in FIG. 23, this embodiment having two pairs of opposing electrodes in accordance with the present invention;
FIG. 26 is a cross-sectional view of the catheter along lines 26-26 of FIG. 20;
FIG. 27 is a partial side view of the working end of another arrangement of a catheter having a balloon and bendable members with electrodes.
FIG. 28 is a cross-sectional view along lines 28-28 of FIG. 26;
FIG. 29 shows a partial cross-sectional view of the venous system of the hemorrhoid region which is to be treated by using the present invention;
FIGS. 30a, 30b, 30c, and 30d are side views of an embodiment of a catheter treating a venous treatment site within a dilated vein in accordance with the present invention.
FIG. 31 is a partial profile view of the anatomical region of the esophageal region, including a vein to be treated by using the present invention;
FIGS. 32a, 32b and 32c are side views of an embodiment of the catheter constructed and delivered to a venous treatment site within a dilated vein for treatment by using the present invention.

### DETAILED DESCRIPTION OF THE EMBODIMENTS

As shown in the exemplary drawings, the invention is directed toward the intravenous treatment of veins using a catheter to deliver at least one electrode to a venous treatment site. As used herein, like reference numerals will designate similar elements in the various embodiments of the present invention to be discussed. In addition, unless otherwise noted, the term working end will refer to the direction toward the treatment site in the patient, and the term connecting end will refer to the direction away from the treatment site in the patient. The invention will be described in relation to the treatment of the venous system of the lower limbs. It is to be understood, however, that the invention is not limited thereto and may be employed intraluminally to treat veins in other areas of the body such as hemorrhoids, esophageal varices, and venous-drainage-impotence of the penis. Furthermore, although the invention will be described as using RF energy from the electrode, it is to be understood that other forms of energy such as microwaves, ultrasound, direct current, circulating heated fluid, radiant light, and lasers can be used, and that the thermal energy generated from a resistive coil or curie point element may be used as well.

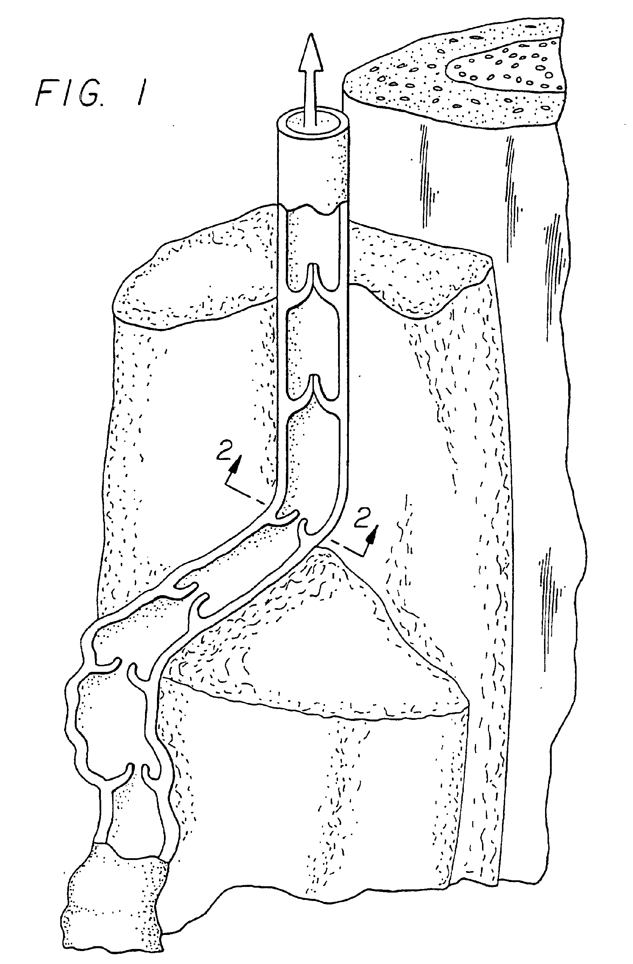

A partial cross-sectional view of a dilated vein from a lower limb having incompetent valves is shown in FIG. 1. These veins are often disposed within muscle tissue. Veins have bicuspid valves, and in a normal and competent valve, each cusp forms a sack or reservoir for blood which, under pressure, forces the free edges of the cusps together to prevent retrograde flow of the blood and allow only antegrade flow to the heart. The arrow leading out the top of the vein represents the antegrade flow of blood back to the heart. The venous valves prevent retrograde flow as blood is pushed forward through the vein lumen and back to the heart.

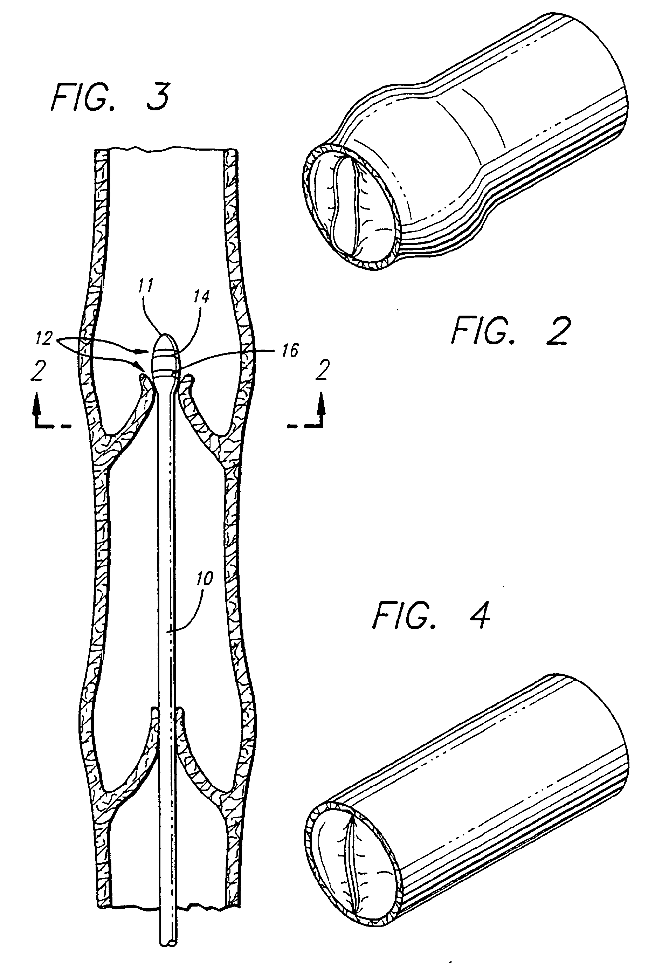

When an incompetent valve encounters retrograde flow, the valve is unable to close, the cusps do not seal properly and retrograde flow of blood may occur. Incompetent valves may result from the stretching of dilated veins. As the valves fail, increased pressure is imposed on the lower veins and the lower valves of the vein, which in turn exacerbates the failure of these lower valves. A cross-sectional perspective view of a dilated vein taken along lines 2-2 of FIG. 1 is illustrated in FIG. 2. The valve cusps can experience some separation at the commissure due to the thinning and stretching of the vein wall at the cusps.

The method of use at the present invention for the minimally invasive treatment of venous insufficiency can be performed using a catheter 10 to deliver electrodes 12 to a venous treatment site in order to restore the competency of a vein. One arrangement of a catheter 10 for delivering the electrodes 12 to the venous treatment site is shown in FIG. 3. The electrodes 12 may be two RF ring electrodes 14 and 16 located at the working end 11 of the catheter 10. This and other arrangements of the catheter 10 will be described in greater detail later. Further, the method is contemplated to be used with any suitable appliance for applying radiant energy, thermal energy, or other forms of energy to heat and shrink the venous tissue in the repair or reconfiguration of incompetent veins in order to restore venous function or valvular competency. Particular discussion will be directed to the treatment of incompetent and varicose veins in the legs, although the method of the present invention is well suited to treating veins in other areas of the body.

When treating the veins of the lower limbs, the patient is typically placed onto a procedure table with the feet dependent in order to fill the veins of the leg. The leg of the patient is prepped with antiseptic solution. A percutaneous introducer is inserted into the vein using the well-known Seldinger technique to access the saphenous or deep vein system. Alternatively, a venous cut-down can be used to access the vein to be treated. The procedure for the repair of incompetent veins can be accomplished by a qualified physician with or without fluoroscopic or ultrasonic observation, or under direct visualization. Further, the physician could palpate the treatment area to determine the location of the catheter, and the treatment site, during the procedure when treating the superficial venous system.

The catheter 10 could be passed within the vein after insertion through the introducer, and advanced through to the venous treatment site. Alternatively, a guide wire for the catheter may be inserted into the vein. The wire is advanced antegrade to the venous treatment site, such as the level of the most proximal incompetent vein site which is to be repaired. The catheter is then inserted upon the wire and is fed up the leg through the vein to the level of the venous section where retrograde flow exists. In either case, the catheter 10 delivers the electrodes 12 to the venous treatment site. Fluoroscopy, x-ray, ultrasound, or a similar imaging technique could then be used to direct the specific placement of the catheter and confirmation of position within the vein. X-ray contrast material can be injected through or around the catheter to identify the incompetent venous sections to be repaired.

From the antegrade approach, the catheter can be pushed through the venous valve so that the electrodes are positioned across the valve of the incompetent venous section to be treated. The catheter 10 travels antegrade through the venous valves, as shown in FIG. 3, and is positioned so that the electrodes 12 are near a dilated section of the vein to be treated. The electrodes may be positioned so as to extend past the incompetent venous valve. When the electrodes 12 of the catheter 10 are positioned at the venous treatment site, the RF generator is activated to provide suitable RF energy, preferably at a selected frequency from a range of 250 kHz to 350 MHZ. One suitable frequency is 40 Mhz. One criteria for the selection of the applied frequency is the minimization of coagulation in the vein. Another criteria is to control the spread and depth of the thermal effect in the tissue. The extent of heating or depth of penetration into the tissue generally increases with lower frequencies, and decreases as the frequency increases. A microprocessor can be used to select a frequency for treating different veins according to the above criteria. For example, the microprocessor can include a table stored in memory for associating specific frequencies for treating various veins and vein diameters according to the criteria of minimizing coagulation and controlling the spread or depth of the heating effect. The energy emitted from the electrodes is converted within the venous tissue into heat. As the temperature of the venous tissue increases, the venous tissue begins to shrink. The shrinkage is due in part to dehydration and the structural transfiguration of the collagen fibers in the vein. Although the collagen becomes compacted during this process, the collagen still retains some elasticity. When RF energy is applied near the locus of the dilated vein and venous valve, shrinkage of the vein can restore valvular competency by reducing the dilation which is preventing the proper functioning of the venous valve.

The working end 11 of the catheter 10 near the electrodes 12 physically limits the amount of shrinkage. The working end 11 is preferably sufficiently sized or enlarged to prevent the complete ligation of the vein. Other schemes, such as an inflatable balloon, may be used to mechanically limit or control the amount of shrinkage in the vein.

Vein dilation is reduced after RF energy applied from the electrodes 12 heats the surrounding venous tissue to cause shrinkage. RF energy is no longer applied after there has been sufficient shrinkage of the vein to alleviate the dilation of the vein near the valves, so as to restore venous function or valvular competency. Sufficient shrinkage may be detected by fluoroscopy, external ultrasound scanning, intravascular ultrasound scanning, impedance monitoring, temperature monitoring, direct visualization using an angioscope, or any other suitable method. For example, the catheter 10 can be configured to deliver x-ray contrast medium to allow visualization by fluoroscopy for assessing the condition of the vein and the relationship of the catheter to the treatment area of the vein during the shrinkage process. As an alternative to fluoroscopy, external ultrasound techniques such as B-scanning using distinct ultrasound signals from different angles, or intravascular ultrasound can be used to acquire a more multidimensional view of the vein shrinkage at the treatment site, which improves the detection of uneven shrinkage in the vein. An angioscope may also be used to directly visualize and determine the extent and degree of vein shrinkage.

After treatment, the commissure and the cusps of the venous valves should be closer together with little separation or prolapse, which indicates a restoration of the competency of the valve. A cross-sectional view of the venous valve after being treated with RF energy is shown in FIG. 4. Valvular competence may be determined by contrast injection or Doppler probe measurement.

Substantial shrinkage may be achieved very rapidly, depending upon the specific treatment conditions. Because the shrinkage can proceed at a rather rapid rate, the RF energy is preferably applied at low power levels. As previously discussed, the frequency of the RF energy is selected to minimize coagulation and to control the spread of the heating effect at the treatment site. The properties of the treatment site, such as temperature, can be monitored to provide feedback control for the RF energy in order to minimize coagulation. Other techniques such as impedance monitoring, and ultrasonic pulse echoing, can be utilized in an automated system which shuts down the application of RF energy from the electrodes to the venous section when sufficient shrinkage of the vein is detected and to avoid overheating or cauterization of the vein. Monitoring these values in an automatic feedback control system for the RF energy can also be used to control the spread, including the depth, of the heating effect. In all instances, the application of RF energy is controlled so as to shrink the venous tissue sufficiently to restore and maintain the competency of the venous valve.

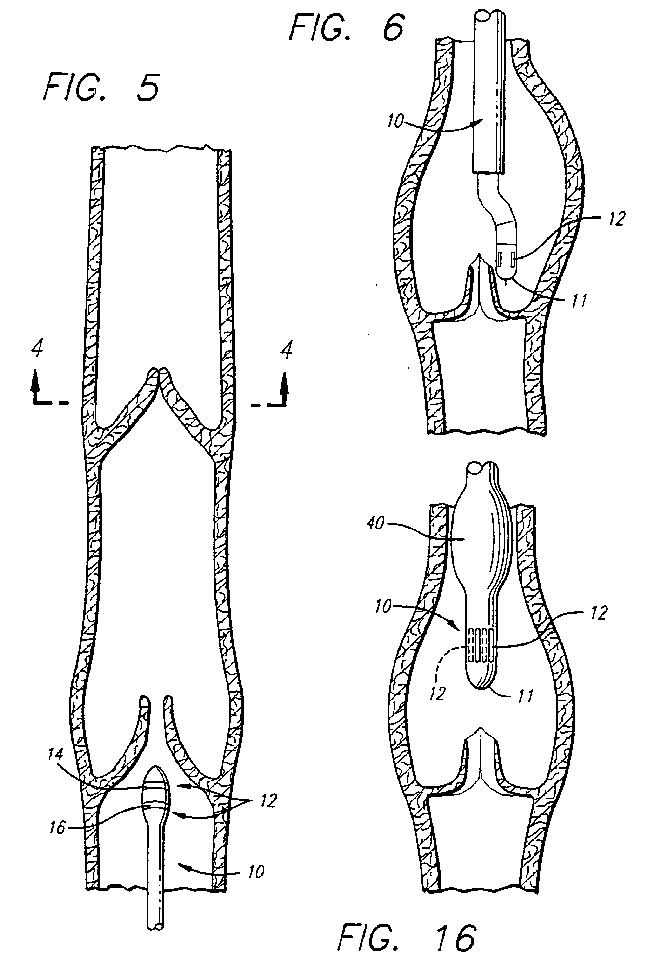

After treating the venous section shown in FIG. 3, the catheter 10 is moved to the next lower venous valve suffering from insufficiency as shown in FIG. 5. The electrode 12 may be placed across the venous valve as discussed previously in connection with FIG. 3. However, an alternative placement of the electrode 12 may be used. For example, as shown in FIG. 5, the electrode 12 is positioned just below or retrograde to the cusps of the venous valve. Placement of the electrode below the valve when applying RF energy can be advantageous in minimizing the effect of localized RF heating on the thin cusps of the venous valve while still achieving shrinkage of the vein to restore venous function or valve competency.

Where the catheter is designed with a fluid delivery lumen, a cooling fluid can be delivered through the delivery lumen to the bloodstream during RF heating of the vein being treated. The delivered cooling fluid minimizes any heating effect on the blood, and reduces the risk of heating the blood to the point of coagulation. The fluid may be delivered through ports formed along the side of the catheter near the working end and the electrodes.

While the method has thus far been described as restoring valvular competency, it is not so limited. A contiguous axial section of dilated vein can be treated by applying RF energy along the dilated venous section, even if the section is extensive. The dilated vein is shrunk and reduced to a normal diameter under the controlled application of RF energy in accordance with the present invention. Such treatment can be used in the cosmetic treatment of varicose veins. Further, thickening of the vein may occur during treatment, which can reduce the likelihood of the recurrence of varicose veins and venous insufficiency.

The catheter 10 can be repositioned to treat as many venous sections and valves as necessary. RF energy is applied to each venous section to be repaired, until all of the desired venous sections are repaired and the valves are rendered competent. Multiple incompetent valves and insufficient or dilated venous sections may be treated and repaired in a single minimally invasive procedure. If desired, a second introducer can be inserted into the limb of a patient in order to access either the deep or the superficial vein system, whichever has yet to be treated. The catheter can then be used to treat incompetent venous sections in the other vein system.

Instead of the antegrade approach, as shown in FIGS. 3 and 5, the catheter can deliver the electrodes to the venous treatment site from a retrograde direction. The catheter 10 is introduced through the skin and into the vein in a retrograde direction. The catheter 10 can penetrate the vein above and adjacent to the incompetent venous section to be treated. The electrodes are advanced until contact with the cusp of the venous valve is observed by fluoroscopy, ultrasound, or other detection method. The catheter is then pulled back slightly to allow treatment of the dilated section of vein. The electrodes are activated to deliver RF energy to the venous tissue and shrink the vein. The shrinkage of the vein can be limited to prevent ligation and allow the continued function of the vein. The outer diameter of the catheter or an extendable member can be controlled to limit the magnitude of the vein shrinkage.

More specific application of the RF energy to the separating commissures of venous valves can be effective in restoring venous function and valvular competency. The catheter 10 can be configured to position the electrodes within the vein and to appose the electrodes with the venous section to be repaired. The catheter is capable of being deflected, torqued, or otherwise moved to allow for proper placement of the electrode. Alternatively, a permanent bend may be formed near the working end of the catheter, which can then be turned and twisted in order to achieve the desired apposition. Manipulating the working end of the catheter enables preferential heating along the vein wall being treated, if desired, where the electrodes are placed closer to one side of the vein wall.

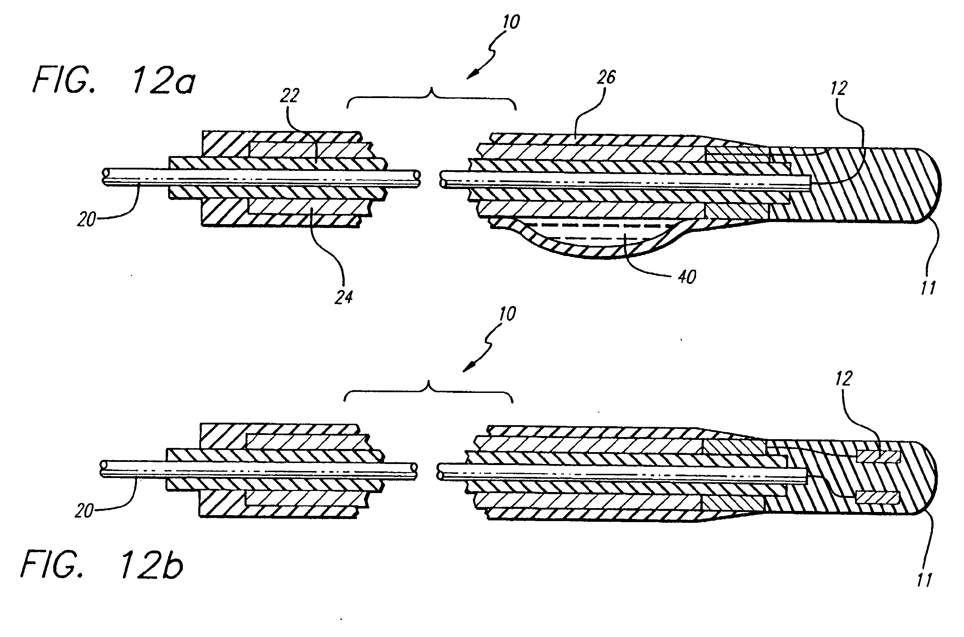

The electrodes 12 on a deflected catheter, as shown in FIG. 6, can be placed in close apposition to the vein walls near the commissure from a retrograde approach. The catheter may also be manipulated to place the electrodes in close apposition to the commissures of the venous valve to cause local shrinkage near the commissures to remedy any separation of the commissures from vein dilation and to restore venous function and valvular competency. After treating one end of the valvular commissure, the catheter may then be moved to place the electrodes near the commissure at the opposite end of the valve. Thus, after selectively applying RF energy to one side of the vein wall, the catheter can be turned 180 degrees around to apply energy to the other side of the vein wall, so as to promote the restoration of the function of the vein. Alternatively, an asymmetrical balloon as shown in FIG. 12, or another such positioning device, can be used to appose the electrodes against the venous section to be treated. The balloon may be deflated and then inflated to allow easier movement and repositioning of the catheter.

After treating one section of the vein, the catheter can be moved to the level of the next section of vein to be repaired. The same procedure would then be repeated for each subsequent instance of vein repair. The treatment may be repeated several times until sufficient shrinkage is achieved to restore venous function and valvular competence, while the vein retains patency. After completing the treatment for the incompetent venous sections, the electrode containing catheter is removed from the vein.

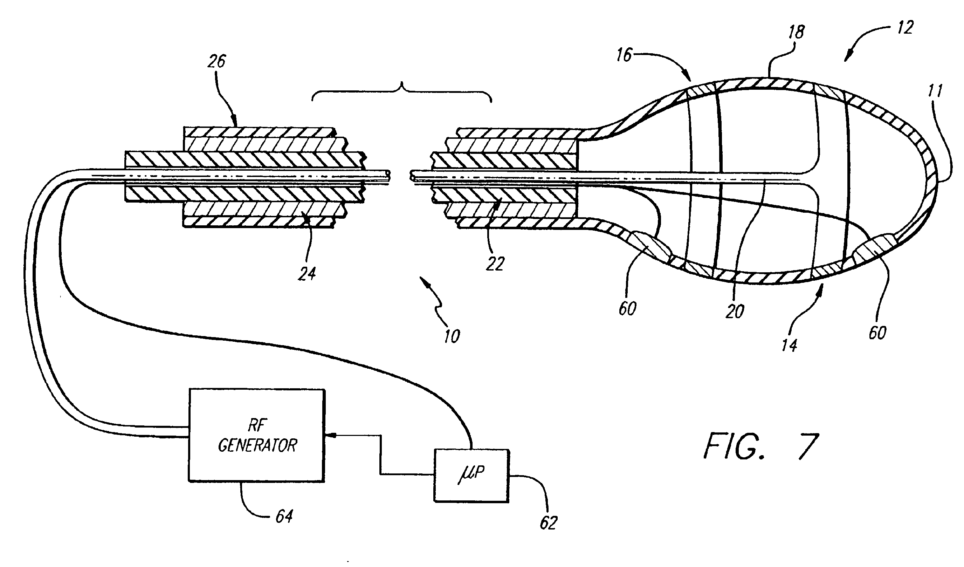

An arrangement of the catheter 10 having electrodes 12 on the working end 11 which causes localized heating of the surrounding venous tissue and shrinkage of the vein described, as shown on FIGS. 3 and 5, is shown in more detail in FIG. 7. The electrodes 12 include two ring electrodes 14 and 16. The end ring electrode 14 can act as the active electrode, and the ring electrode 16 can act as the return electrode, or vice versa. The end ring electrode 14 is preferably spaced away from the tip of the working end of the catheter which may be formed from plastic or some other non-conductive material. The RF field created by the ring electrodes 14 and 16 should not extend past the end of the catheter. The inert non-conductive tip of the working end of the catheter helps prevent shrinkage past the end of the catheter by limiting the extent and formation of the RF field. This non-conductive tip acts as a shrink-limiting mandrel to prevent the veins from shrinkage to a diameter less than the catheter tip and can extend 2 to 25 mm past the electrode 14. Both electrodes 14 and 16 are preferably made from stainless steel. An insulator material 18 is located between the end electrode and the ring electrode. The catheter 10 and electrodes 12 should be constructed from materials which would allow visualization under fluoroscopy, x-ray, ultrasound, or other imaging techniques. For example, the catheter 10 can be configured to deliver x-ray contrast medium to allow visualization by fluoroscopy. Contrast media injected into the vein can be used to assess the condition of the vein and the relationship of the catheter to the treatment area of the vein by phlebography during the shrinkage process.

The catheter 10 includes a stranded, twisted center conductor 20 surrounded by a layer of insulation 22 which is preferably formed from TFE Teflon^{®}. A silver-coated copper braid 24 surrounds the insulated center conductor, and provides flexible and torqueable characteristics to the catheter shaft. A sheath 26 covers the copper braid 24. The sheath 26 is preferably made of an electrically resistive, biocompatible material with a low coefficient of friction such as Teflon^{®}. The center conductor 20 is connected to a power source 64 such as an RF generator, to provide RF energy to the electrodes 12.

While the electrodes 12 have been described as ring electrodes, other electrode configurations and arrangements can be used. For example, equidistantly spaced longitudinal electrodes can be used to provide omnidirectional and circumferential shrinkage and to minimize lengthwise contraction of the vein. The electrodes form an RF field circumferentially around the electrode.

It is to be understood that although a bipolar arrangement is described, a monopolar arrangement may also be used. In a monopolar arrangement, an inside electrode, such as a mesh or wire electrode, is inserted into a cavity in a patient's body. An outer electrode having a much larger surface area than the inside electrode is placed on the outer surface of the patient's body near the treatment site. For example, an external metal plate is placed on the skin over the region to be treated by the inside electrode. The electrodes are connected to a RF generator which produces an electric field within the patient's body. Because the surface area of the inner electrode is much smaller than that of the outer electrode, the density of the electric field is much higher around the inside electrode. The electric field reaches its highest density between the two electrodes in the region near the inside electrode. The increased density of the field around the inside electrode allows localized heating of the tissues surrounding the inside electrode. The degree of heating may be dependent on such factors as the impedance and dielectric constant of the tissue being heated.

The end ring electrode 14 and the ring electrode 16 are preferably located between the sensors 60 for measuring values such as impedance. In measuring impedance, as will be described in further detail later, the area between the electrodes often provides the most relevant data. It is to be understood that the sensors 60 may be used to measure other values including temperature and ultrasound signals. Further, the positioning of the sensors 60 on the catheter 10 can vary depending on the value being measured. For example, when measuring temperature, it may be desirable to place the sensor on or immediately adjacent to the electrode. The temperature sensor can sense the temperature of the tissue around the electrodes. When measuring echo signals of pulsed ultrasound, the sensors may be placed between the electrodes, or at the tip of the catheter. When measuring pulse echo ultrasound signals, the catheter is preferably rotated to resolve an image of the environment surrounding the catheter and the sensors.

The sensors 60 measure parameters which can be used to determine the extent of vein shrinkage. For example, the sensors 60 can be sensing electrodes which measure the impedance of the venous tissue in contact between the end electrode 14 and the ring electrode 16. A constant RF current is emitted from the active end electrode 14 to the return ring electrode 16. Also, the impedance may be measured between the electrodes 14 and 16 directly. The voltage across the electrodes is measured by the sensing electrodes to detect the impedance of the volume between the electrodes. The voltage measured is proportional to the impedance Z between the electrodes, where Z = V/I and the current, I, is constant. The impedance changes as a function of the diameter of the vein because there is less blood and less conductance as the venous diameter decreases. As the volume decreases due to shrinkage, the amount of conductive volume between the electrodes decreases, and the increased impedance causes a corresponding increase in the measured voltage. This technique allows for the measurement of vein shrinkage in relative terms. The signals from the sensing electrodes can be input to a monitor, or microprocessor 62 which could send control signals to the RF generator 64 in order to control the application of RF energy to the electrodes in accordance with the relative impedance measured. Alternatively, the signals from the sensing electrodes can be displayed visually on a monitor in order to allow for manual control by the physician.

In an alternate arrangement, the sensors 60 can instead be temperature sensors such as thermistors. The temperature sensors may be included on the catheter near the electrodes on the working end to monitor the temperature surrounding the electrodes and the venous section being treated. Application of RF energy from the electrodes may be halted when the monitored temperature reaches or exceeds the specific temperature at which venous tissue begins to shrink. The signals from the temperature sensors can be input to the microprocessor 62 for controlling the application of RF energy to the electrodes in accordance with the monitored temperature.

Instead of sensing electrodes or thermistors, another arrangement includes ultrasonic piezoelectric elements which emit pulsed ultrasound waves as the sensors 30. The piezoelectric elements are operated in a pulse-echo manner to measure the distance to the vein wall from the catheter shaft. Again, the signals representative of the pulse-echo would be input to the microprocessor 62, or to a monitor to allow for manual control, and the application of RF energy would be controlled in accordance with the distance computed between the catheter and the vein wall.

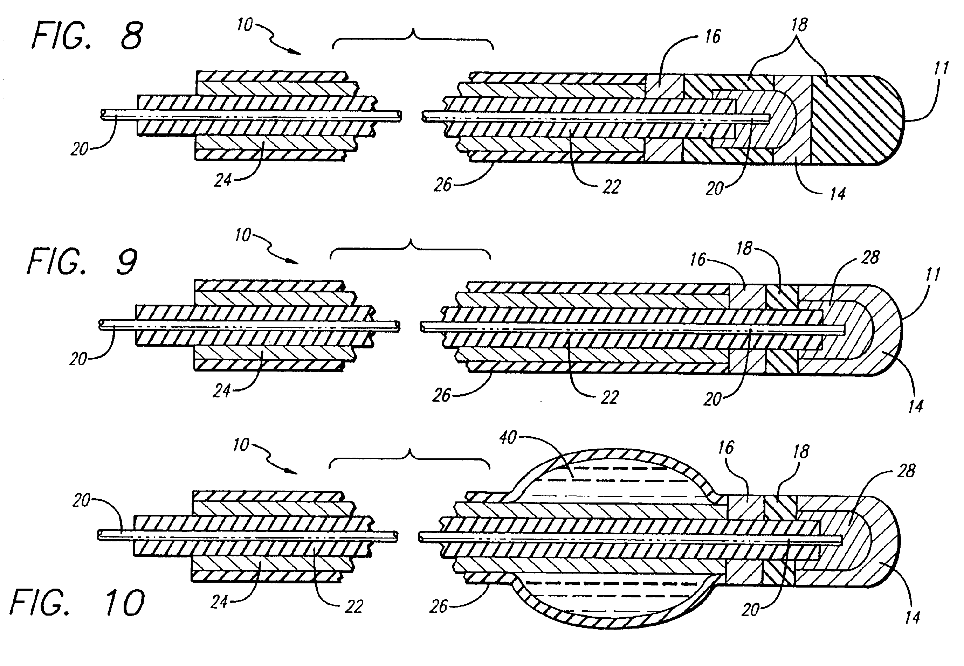

The working end 11 of the catheter 10, as shown in FIG. 7, is rounded to provide an atraumatic tip which minimizes any incidental damage as the catheter is manipulated within the vein. The working end 11 of the catheter 10 can have an enlarged dimension which limits the amount of local vein shrinkage. An enlarged atraumatic tip may be achieved using a bulbous shape for the working end 11. Different sized working ends 11 and electrodes 12 can be manufactured separately from the catheter 10 for later assembly with the shaft of the catheter 10 so that a single catheter shaft may be used with working ends having a variety of diameters. A working end having a specific size or shape could then be used with the catheter 10 depending on the type of vein being treated. For example, certain larger veins have a diameter of seven to eight millimeters (mm), while other veins only have a diameter of 2 to 3.5 mm. Alternatively, the working end 11 and the ring electrodes 14 and 16 can be flush with the shaft of the catheter as shown in FIG. 8. Other methods, such as monitoring the amount of shrinkage by fluoroscopy, may be used to determine and control the amount of shrinkage. In other respects, the construction of the catheter in FIG. 8 is similar to that of FIG. 7, as previously discussed.

Another arrangement of the catheter 10 includes an end electrode 14 which is a cap electrode formed on the tip of the working end 11 of the catheter 10. As shown in FIG. 9, the end electrode 14 is preferably fabricated from stainless steel. The end electrode 14 acts as the active electrode, and the ring electrode 16 acts as the return electrode. The cap electrode 14 of the catheter 10 is rounded to provide an atraumatic tip so as to minimize any damage to the surrounding venous tissue as the catheter is manipulated through the vein. The outer diameters (O.D.) of the electrodes 14 and 16 in one example size is 7 French or about 2.3 mm. Alternatively, the cap electrode and the working end 11 of the catheter 10 may have an enlarged dimension from the remainder of the catheter. The electrodes and the working end, as shown in the exemplary FIG. 9, are substantially flush with the remainder of the catheter. The braid sheath 26 covers the silver-coated, copper braid 24 of the catheter, and the sheath is flush with the outer diameter of the ring electrode 16. An insulator tube 18 is located between the end electrode and the ring electrode. At the working end of the catheter, a solder fill 28 is formed between the center conductor 20 and the end electrode 14. The center conductor 20 is isolated from the ring electrode 16 by insulation 22. The end cap electrode of FIG. 9 does not limit shrinkage of the vein adjacent to the tip of the catheter and therefore can allow the vein to shrink completely if desired.

In another arrangement an inflatable balloon 40 coaxially placed over the braided shaft can center the catheter 10 and the electrodes 14 and 16 within the vein lumen in order to avoid unintended electrode contact with the vein lumen which could otherwise result in uneven heating of portions of the vein lumen. As shown in FIG. 10, the balloon 40 is located adjacent to the electrode 16 which is closer to the connecting end of the catheter. The balloon 40 is preferably expandable and compliant, and fabricated from an elastic material such as latex, which can provide intermediate diameters. The balloon can be inflated with saline or other conductive solutions.

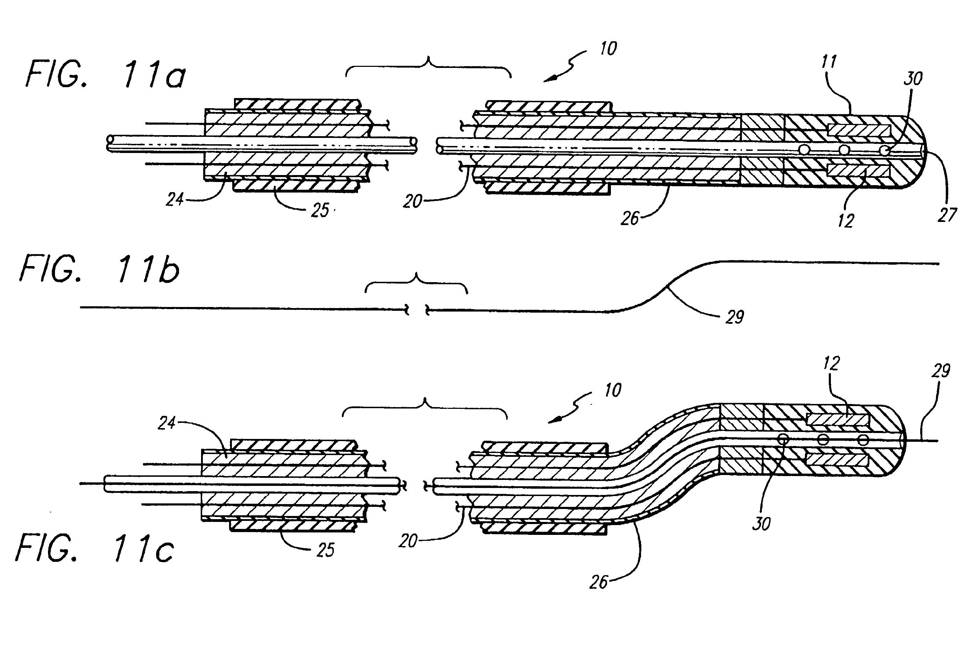

As discussed in connection with FIG. 6, it can be desirable to maintain selective apposition between the electrodes and the venous tissue at the treatment site. An arrangement of the catheter 10, shown in FIGS. 11a, 11b and 11c, is capable of being deflected by a shaft deflection wire 29. The catheter includes a silver-coated copper shield 24 and an outer layer of insulation 26. The electrodes 12 can be four circumferentially spaced longitudinal electrodes, as previously discussed. FIGS. 11a and 11c only show two of four longitudinal electrodes. The catheter 10 further includes a stiffening jacket 25 formed around the catheter shaft, except for the working end of the catheter. A central hollow wire lumen 27 extends through the length of the catheter. The shaft deflection wire 29 has a stiff bend formed near its working end, and is pushed through the wire lumen 27 of the catheter. The end of the wire 29 after the stiff bend which advances through to the tip of the working end of the catheter is preferably flexible and pliant. The stiffening jacket 25 prevents the catheter shaft from being deflected by the shaft deflection wire 29 until the deflection wire reaches the working end of the catheter. The bend in the deflection wire 29 moves the working end 11 of the catheter to one side. The electrodes 12 can then be selectively placed in apposition with the specific venous tissue to be treated. A contrast medium can also be administered to the treatment site through the lumen 27. Further, a cooling solution or fluid may be delivered to the treatment site through the lumen 27. Side ports 30 for the lumen can be formed at the working end near the electrodes 12 for delivering the contrast medium and the cooling fluid. Alternatively, the lumen 27 could be closed at the tip of the working end of the catheter in order to allow an injection of contrast media or cooling solution to be forced out the side ports 30. Closing the lumen 27 at the tip further allows the deflection wire 29 to be made more stiff without concern for the stiffer wire extending past the catheter.

Another arrangement uses an asymmetrical balloon 40 to deflect the electrodes 12 at the working end 11 of the catheter to one side. The electrodes 12 are a pair of longitudinal electrodes located on one side of the catheter. As shown in FIGS. 12a and 12b, the balloon 40 is located on the opposite side of the catheter. When the balloon 40 is inflated, the opposite side of the working end 11 accommodating the longitudinal electrodes is moved into apposition with the venous tissue to be treated. After treating the dilated venous section, the balloon 40 can be deflated, and the catheter removed from the vasculature. It should be noted that the other mechanisms for deflecting the working end of the catheter may be used. For example, a bendable actuation wire may be used on one side of the catheter in order to perform a function similar to that of the asymmetrical balloon. The catheter further includes the jacket 26, the braid 24, and the TFE insulation 22, and is similar in construction to the previously discussed embodiments.

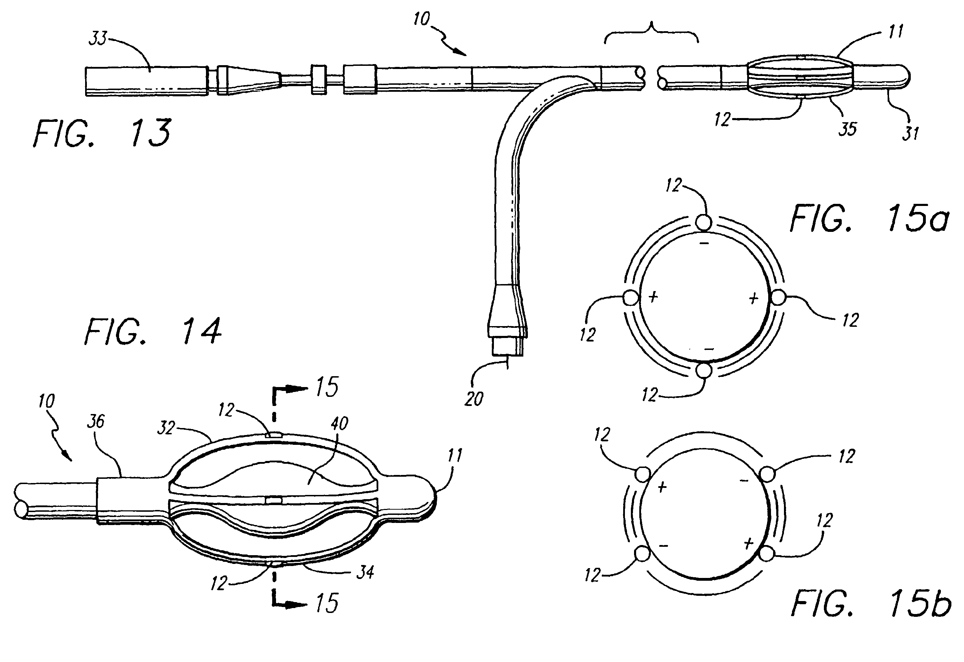

In an embodiment of the present invention, as shown in FIG. 13, the catheter 10 includes bowable electrodes 12 in the form of four conductive elongate members. The bowable electrodes 12 are similar to longitudinal electrodes formed along the circumference of the catheter, but are not fixed to the catheter. The catheter itself can fit through a suitably sized sheath for the procedure. For example, a 9 French sheath, which has about a 3 mm diameter, may be used. The working end 11 of the catheter includes a moveable tip 31 manually controlled by a diameter actuator 33 located at the connecting end of the catheter. The movable tip 31 is connected to the diameter actuator 33 by a central wire (not shown) running through the catheter. The diameter actuator 33 may be threaded onto the connecting end of the catheter. Maneuvering the actuator 33 into and out of the connecting end of the catheter causes a corresponding movement in the movable tip 31 at the working end of the catheter. If the movable tip 31 is pulled toward the connecting end by the diameter actuator 33, then the electrodes 12 are bowed outwardly. The bowable electrodes 12 preferably expand out to treat veins up to 8 mm. If the movable tip 31 is pushed out by the diameter actuator 33, the bowable electrodes 12 are then retracted towards the shaft of the catheter. Consistent contact of the electrode can be maintained with the vein wall.

The extent of shrinkage can be controlled by the effective diameter of the catheter and the electrode combination. The electrodes 12 may be bowed radially outwards as part of the effective diameter of the catheter so as to come into apposition with the vein wall. As RF energy is applied, the vein begins to shrink down to the effective diameter of the catheter. The effective diameter of the catheter is reduced under the control of the physician to control the amount of shrinkage. As the effective diameter is decreased, the electrodes continue to maintain apposition with the venous tissue. As before, the extent of vein shrinkage can be monitored by fluoroscopy, or any other suitable method. After shrinking the vein to the desired diameter, the application of RF energy from the electrodes 12 is ceased. The desired diameter can be the final effective diameter of the catheter, as defined by the deflected electrodes 12.

The electrodes 12 may be fabricated from spring steel or nitinol so that the electrodes 12 would be biased to return to a reduced diameter profile. Where the entire length of the bowable longitudinal electrode is conductive, insulation 35 may be provided over the majority of the electrode surface in order to prevent any unintended heating effects. The ends of the electrodes are insulated from each other to prevent creating variable field densities at the ends, especially as the effective diameter increases which would create even greater field disparities between the ends and the bowed midsection. The insulation 35 can be polyimide or another type of insulating film. Insulation 35 provided along the back of the electrodes away from the vein wall further prevents heating of the blood flowing in the vein, which should also reduce the likelihood of coagulation. The remaining exposed area of the electrode is preferably the area which contacts the vein wall during apposition. The heating effect is then focused along the vein wall. The exposed surface area of the electrode should be as great as allowable while maintaining a consistent distance between the exposed sections of the electrode along the circumference of the effective diameter. The larger the exposed surface of the electrodes apposed against the vein wall during shrinkage, the greater the surface area of the vein wall affected by the electric field generated by the electrodes.

Another arrangement of the catheter 10, as shown in FIG. 14, includes bowable elongate members 32 having one end anchored to the working end 11 of the catheter, and the other end slidably connected to the catheter towards the connecting end. The catheter shown in FIG. 14 is similar to that shown in FIG. 13, except that instead of having the elongate members act as the electrodes themselves, the electrodes 12 are located on the elongate members 32. The elongate members 32 preferably include a flat central area 34 for the electrodes 12. The central area 34 remains substantially flat as the elongate members 32 are deflected and bowed outwardly. The substantially flat central area allows for a more uniform contact with the vein wall. The flat area establishes a larger surface area to assure contact between the electrode 12 on the elongate member and the vein wall. It is to be understood that the flat area 34 need not be centrally located on the elongate member 32. The flat area should be located so as to be the first area that contacts the vein wall. The elongate members 32 shown in FIG. 14 are connected to a sliding sleeve 36 formed along the exterior of the catheter shaft. As the electrodes 12 are moved radially outwards and inwards, the slidable sleeve 36 is moved towards and away from the working end.

The balloon 40 can be furnished between the catheter shaft, and the elongate members 32. Manual manipulation of the sliding sleeve is not required in this arrangement, and the sleeve need not travel any substantial length of the catheter. The balloon 40 is inflated and comes into contact with the elongate members 32. As the balloon 40 is further inflated, the electrodes 12 are moved outwardly in a radial direction as the elongate members are deflected and bowed by the expanding balloon 40. The balloon is preferably inflated using a non-conductive fluid, especially where the elongate members contain the electrodes, or where the elongate member itself is conductive so as to act as the electrode. When the proper diameter for the electrodes is reached, the inflation of the balloon ceases, and the application of the RF energy begins. The balloon 40 covers a greater surface area over the venous treatment site, and ensures proper electrode placement relative to the vein wall while controlling the amount of venous shrinkage. More precise control over the shape and diameter of the balloon can also be possible using the bowable members. As RF energy is applied, the vein begins to shrink down. The effective diameter of the catheter is reduced under the control of the physician to control the amount of shrinkage. As the effective diameter is decreased, the electrodes continue to maintain apposition with the venous tissue. The application of RF energy from the electrodes 12 is terminated after shrinking the vein to the desired diameter, which is the final effective diameter as defined by the diameter of the balloon 40 and the deflected elongate members 32. The balloon 40 is then is deflated to 2 minimal profile. The elongate members 32 are preferably fabricated from spring steel or nitinol so that the elongate members 32 would be biased to return to a reduced diameter profile when the balloon is deflated.

A cross-sectional view of the electrodes 12 of FIG. 14 along lines 15-15 is shown in FIG. 15a. In the four-electrode configuration, a preferred arrangement is to have the electrodes 12 spaced equidistantly apart along the circumference of the catheter. The polarity of each electrode is preferably opposite to the polarity of the immediately adjacent electrodes. Thus, a uniform RF field would be created along the circumference of the catheter by the alternating electrodes. In another arrangement as shown in FIG. 15b, if adjacent electrodes were to be moved closer together, two effective pairs of active electrodes of opposite polarity would be formed along the circumference of the catheter. While an RF field would still be formed along the entire circumference of the catheter, the RF field would be strongest between the closest adjacent electrodes of opposite polarity. Shrinkage of the vein would be concentrated where the RF field was strongest.

In an alternative arrangement of that discussed in connection with FIG. 14, the outer sleeve 36 can extend down the length of the catheter to allow the operator or physician to mechanically control the effective electrode diameter during the application of RF energy, so that a separate balloon 40 is not required. Moving the slidable sleeve toward the working end 11 of the catheter causes the electrodes to deflect and radially bow outward to an increased diameter. The outer sleeve 36 can be moved a preset distance to cause the electrodes to bow outwardly to a known diameter. Bowing the electrodes outwardly also places the electrodes in apposition with the venous tissue to be treated. Moving the sleeve 36 toward the connecting end of the catheter pulls back and flattens the electrodes against the catheter before insertion or withdrawal from the vein. Moving the sleeve controls the diameter of the electrode deployment for proper treatment of vein lumen having different diameters, and for providing varying degrees of vein shrinkage. For example, the electrodes could be placed in contact with the venous tissue, and the effective diameter could be mechanically reduced to control shrinkage while RF energy was being applied.

In another arrangement, instead of an outer sleeve, the ends of the elongate members that would otherwise be attached to the outer sleeve are instead slidably located within longitudinal slots or channels disposed along the circumference of the catheter. The ends of the bowable members would slide towards the working end within these channels as the members are deflected or bowed outwardly, and retreat back towards the connecting end in order to return to their original configuration.

In another alternate arrangement the electrodes and the elongate members could be replaced by a single wire mesh or braided electrode, preferably when applying RF energy in a monopolar configuration. As before, the balloon could radially extend the mesh electrode outward into apposition with the vein wall. The balloon can also control the amount of vein shrinkage.

An alternative method for changing the effective diameter of the catheters in FIGS. 13 and 14 is to move the electrodes 12 into direct contact with the vein wall. As the electrodes emit RF energy, the vein wall shrinks and pushes the electrodes inwardly towards the catheter. The vein shrinkage reduces the effective diameter directly, rather than by the active control of the physician, thereby eliminating the need for constant fine mechanical adjustments to the effective diameter. A mechanism such as a push rod or fixed-diameter balloon can be included to prevent further radial contraction of the electrodes at a specific effective diameter, thereby controlling and limiting the amount of vein shrinkage. This has the advantage of maintaining the electrodes in apposition with the venous tissue so that the tissue is heated more than the surrounding blood, without requiring the physician to constantly adjust the effective diameter of the catheter while applying the RF energy.

Other devices which are controllably expandable or extendable can be used to limit the shrinkage of the vein to a desired size. For example, mandrels can be advanced out through the sides of the catheter to define a diameter limit for shrinking the venous section. As another example, a bowable conductive deflection wire can be located on one side of the catheter for achieving apposition with the vein wall. Furthermore, even the non-expandable catheter shaft and electrode shown in FIG. 7 can be used to limit the amount of vein shrinkage during the procedure. The vein would merely shrink down to the fixed diameter of the catheter.

Other methods may be used with the catheter for maintaining apposition. For example, a pressure cuff may be used to apply external pressure to the leg to compress the treatment area so that the vein wall comes into contact with the electrodes. Apposition of the electrodes with the venous tissue would be maintained by the applied external pressure. Such external compression may be used when treating the superficial veins. Methods other than the aforementioned mechanical methods may also be used to control the magnitude of vein shrinkage. Such non-mechanical methods include controlling the time and temperature of the venous RF treatment.

The working end of the catheter 10 could be constructed to have a bend near the working end as shown in FIG. 11 so that the catheter can be rotated to create a stirring effect within the vein in order to achieve more uniform heating of the venous tissue for more even shrinkage. Rather than a permanent bend, the catheter can be manufactured to provide a controllable bend near the working end. For example, the bend may be formed from a shape-memory metal, manipulatable by a system of wires, a torquable braid, or a permanent bend in the catheter.

Another method for controlling the heat transfer to achieve more uniform heating is by using an external tourniquet to reduce blood flow or compress the vein around the catheter at the venous treatment site. By reducing blood flow either by external compression or an intravenously inflated occlusive balloon, the influence of blood flow through the vein, which can carry heat away from the treatment site, is minimized. The heat transfer to the venous tissue during the procedure is less impacted by the blood flow, and the shrinkage rate of the vein would therefore be more predictable. Sufficient pressure may also be established by the external tourniquet to cause the vein to come into apposition with the electrodes.

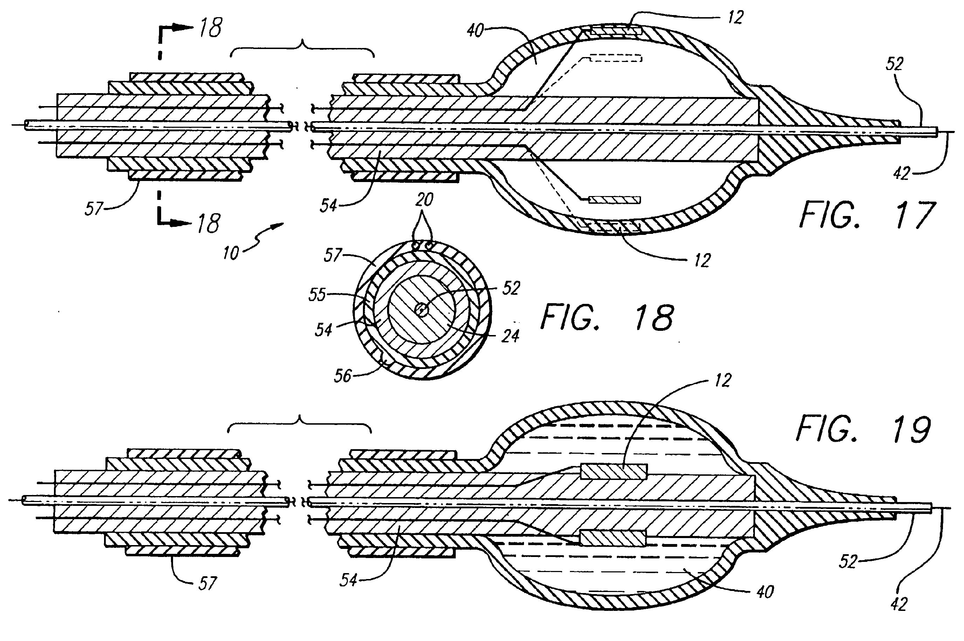

In another arrangement as shown in FIG. 16, an occlusive centering balloon 40 is used to retain a static pool of blood near the venous treatment site. A single occlusive balloon 40 may be used in conjunction with the venous valve to retain a pool of blood to be heated, wherein the electrodes 12 are located between the venous valve and the occlusive balloon 40. Two occlusive balloons (not shown) may be formed on either end of the electrodes to create a static pool of blood at a venous treatment site away from the venous valve. Such an arrangement isolates and protects the venous valve when treatment of the valve is not desired. The occlusive balloons may also be used to center the electrode within the vein lumen.

Although not limited to the occlusive balloon arrangement shown in FIG. 16, the catheter 10 further includes the electrodes 12 arranged in longitudinal fashion around the circumference of the catheter. This arrangement is similar to the embodiments disclosed and described in connection with the FIGS. 13 and 14, however, the electrodes in this instance are fixed on the catheter and do not bow outwards. This fixed diameter arrangement allows a RF field to be formed along the circumference of the catheter. Such an arrangement can provide omnidirectional shrinkage and avoid lengthwise contraction of the vein. The particular positioning and orientation of the longitudinal electrodes is preferably as shown in FIG. 15a.

A balloon expandable arrangement as shown in FIG. 17, includes the four longitudinal electrodes 12 arranged in longitudinal fashion around the circumference of the balloon 40 of the catheter 10. This arrangement is similar to the embodiments disclosed and described in connection with FIGS. 13 and 14, so as to provide omnidirectional shrinkage and minimize lengthwise contraction of the vein. The particular positioning and orientation of the longitudinal electrodes is preferably equidistant as shown in FIG. 15a. The catheter 10 as shown in FIG. 17 is an over-the-wire type in which the catheter travels over a guide wire 42 through a guidewire lumen 52. The catheter 10 further includes the braided shield 24 surrounding the guidewire lumen 52. A braid tube 54 is formed around the braid 24. The lumen 56 for the balloon 40, and the balloon tube 55, encircle the braid tube 54. The braid tube forms a sealing barrier against the inflation fluid leaking into the guidewire lumen 52 from the balloon lumen. The exterior of the catheter includes a retainer tube 57 holding the conductor leads 20, which connect the electrodes 12 to an RF generator. A cross-section of the shaft of the catheter 10 along lines 18-18 of FIG. 17 is shown in FIG. 18.

In another arrangement, the electrodes 12 are located under the balloon 40 of the catheter 10. This arrangement, which is shown in FIG. 19 and which is similar to that shown in FIGS. 17 and 18, allows for conductive heating of the venous tissue. The catheter 10 shown in FIG. 19 is an over-the-wire type in which the catheter travels over the previously introduced guide wire 42. The balloon is inflated and expands to come into contact with the venous tissue. As discussed previously, the inflated balloon 40 can be used to control or limit the magnitude of shrinkage of the vein to the outer diameter of the inflated balloon 40. The effective diameter can be controlled by the selective inflation and deflation of the balloon 40. The inflation medium of the balloon 40 is preferably a conductive fluid, such as saline solution, so that a significant amount of the RF energy will still be transferred to the surrounding venous tissue. However, the inflation medium may absorb a certain amount of the RF energy, which will then be converted to heat. This diffusion of the RF energy could provide greater control over the shrinkage of the vein. Alternatively, a conventional heater coil or curie point element could be used in place of the electrodes 12 in order to directly heat the inflation medium, which in turn would conductively transfer the heat to the venous tissue.

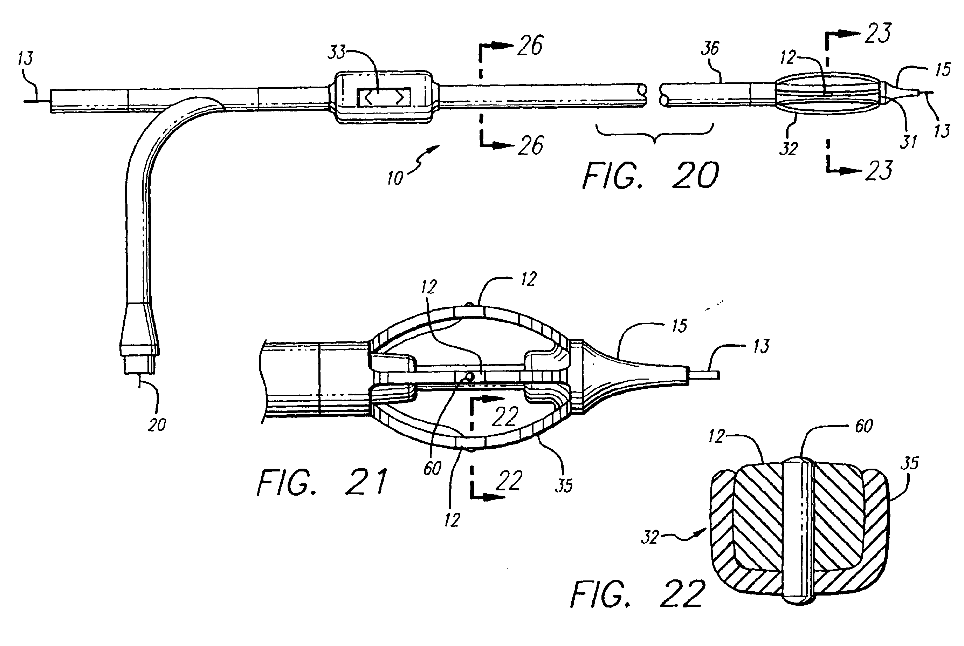

An embodiment of the catheter 10 of the present invention having electrodes 12 on the working end 11 which causes localized heating of the surrounding venous tissue and shrinkage of the vein is shown in FIG. 20. The catheter 10 includes electrodes 12 in the form of four conductive elongate members which can be bowed outward. The bowable electrodes are formed along the circumference of the catheter, but are not fixed to the catheter. The catheter itself is fit through a suitably sized sheath for the procedure. For example, a 7 French sheath, which has about a 2.3 millimeter (mm) diameter, may be used. The sheath is composed of a biocompatible material with a low coefficient of friction. The working end 11 of the catheter includes a tip 15 which is attached to one end of each electrode, and the other end of each electrode is connected to a sliding sleeve 36 formed along the exterior of the catheter shaft. The outer sleeve extends down the length of the catheter to allow the physician to directly and mechanically control the effective electrode diameter during the application of RF energy. As the slidable sleeve 36 is moved towards and away from the working end in response to a control actuator 33, the electrodes 12 are urged radially outwards and inwards, respectively. The tip 15 essentially remains stationary while the slidable sleeve is moved. Moving the sleeve 36 back toward the connecting end of the catheter pulls back and flattens the electrodes against the catheter before insertion or withdrawal from the vein. Moving the sleeve 36 forward toward the working end of the catheter causes the electrodes to deflect and radially bow outward to an increased diameter. The contact area of the electrodes is bowed outwardly as the opposite ends of the longitudinal electrode are moved closer together. The outer sleeve may be moved a preset distance to cause the electrodes to bow outwardly to a known diameter. Bowing the electrodes outwardly also places the electrodes in apposition with the venous tissue to be treated. By manipulating the slidable sleeve to adjust the effective diameter of the catheter defined by the radial bowing of the electrodes, contact between the electrodes and the vein wall can be maintained as the vein shrinks. The control actuator 33 is a switch, lever, threaded control knob, or any other suitable mechanism, preferably one which can provide fine control over the movement of the slidable sleeve. By using the control actuator to move the slidable sleeve, the effective diameter of the electrode can be controlled for treating vein lumen having different diameters, and for providing varying degrees of vein shrinkage.

The tip 15 has a nosecone shape, or can have any shape which allows tracking of the catheter over the guide wire and through bends in the venous vascular system. The nosecone tip can be fabricated from a polymer having a soft durometer, such as 70 Shore A. Alternatively, the nosecone can be constructed from a spring covered with a thin layer of polyethylene shrink tubing.

The extent of shrinkage is controlled by the effective diameter of the catheter and the electrode combination. The electrodes 12 are bowed radially outward as part of the effective diameter of the catheter so as to come into apposition with the vein wall. After being placed in contact with the venous tissue, and the effective diameter could be mechanically reduced to control shrinkage while RF energy was being applied. The electrodes 12 are preferably operated as bipolar electrodes. As RF energy is applied to the electrodes, an RF field is created around the effective diameter of the catheter as defined by the bowed electrodes, and the vein becomes heated and begins to shrink. The effective diameter of the catheter is reduced under the control of the physician to control the amount of shrinkage. As the effective diameter is decreased, the electrodes continue to maintain apposition with the venous tissue. The extent of vein shrinkage is monitored by fluoroscopy, or any other suitable method. After shrinking the vein to the desired diameter, the application of RF energy from the electrodes 12 is ceased. The desired diameter of the vein is the final effective diameter of the catheter, as defined by the deflected electrodes 12.

The electrodes 12 have an elongated shape and may be fabricated from stainless steel, spring steel, or nitinol, so that the electrodes 12 would be biased to return to a reduced diameter profile. The electrodes are rounded wires to facilitate flexing of the catheter at the working end while being delivered through the tenuous venous vasculature. The diameter of the electrodes are preferably between about 0.12 to 0.35 mm (about 0.005 to 0.015 inches), but can be up to about 0.7 mm (about 0.03 inches). Other shapes including rectangular wires having relatively large flat surfaces for contacting the vein wall can be used. Such rectangular wires can have widths ranging from 0.12 mm to 1.2 mm (0.005 to 0.05 inches), and preferably between 0.35 mm to 0.7 mm (0.015 and 0.030 inches), to allow four to eight electrodes around the catheter shaft.

The entire length of the bowable longitudinal electrode is conductive, and insulation 35 is provided over the majority of the electrode surface, as shown in FIGS. 21 and 22, in order to prevent any unintended heating effects. Only a modest portion of the conductive surface is exposed to act as the electrode. The heating effect is greatest when the electrodes are close together since the electrical field density (power density) is greatest at this point. The ends of the electrodes are insulated from each other to prevent creating electrical field densities that are larger at the ends compared to that around the middle of the electrode. As the effective diameter increases, greater field disparities between the ends and the outwardly bowed midsections could be created if no insulation were provided. The insulation 35 can be polyimide, parylene, or another type of insulating material. The insulation 35 provided along the sides and the back of the electrodes opposite from the vein wall further prevents heating of the blood flowing in the vein, which should also reduce the likelihood of coagulation. Where the wire has a rectangular shape, then the exposed area which functionally acts as the electrode would then occupy only one face of that wire. As shown in FIG. 22, the insulation 35 surrounding the electrode can further cover the peripheral edges of the exposed face of the electrode to further isolate the blood flow from unintended heating effects.

The exposed area of the electrode is preferably the area which directly contacts the vein wall during apposition. The heating effect is then focused into the vein wall. The exposed surface area of the electrode should be as great as allowable while maintaining a consistent distance between the exposed sections of the electrode along the circumference of the effective diameter. The larger the exposed surface of the electrodes apposed against the vein wall during shrinkage, the greater the surface area of the vein wall affected by the electric field generated by the electrodes. The exposed area for the electrode can be substantially flat to enhance uniform contact with the vein wall and for controlling the diameter of the vein.

A sensor 60 such as a small thermocouple for measuring temperature is attached to the electrode 12. As shown in the cross-sectional view of FIG. 22, the temperature sensor 60 is soldered in place through a hole in the electrode so that the sensor is nearly or substantially flush with the exposed surface of the electrode. The sensor can accurately sense the temperature of the vein wall in apposition with the exposed electrode surface. The leads to the sensor are situated on the opposite side of the electrode which is insulated.

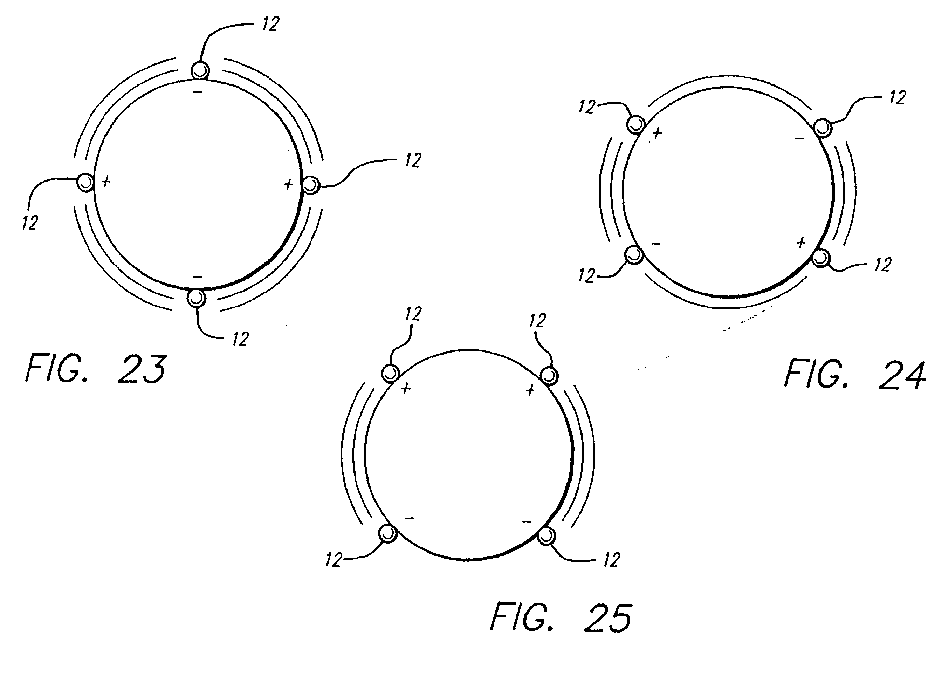

A cross-sectional view of the electrodes 12 of FIG. 20 along lines 23-23 is shown in FIG. 23. In the four-electrode configuration, a preferred embodiment is to have the electrodes 12 spaced equidistantly apart along the circumference of the catheter. Although the catheter has been described as having a four electrode configuration, it is to be understood that the catheter may include a different number of electrodes, for example, six, eight, or more bowable electrodes, in order to lessen the inter-electrode gap and reduce the amount of power required to heat the venous tissue. The polarity of each electrode is preferably opposite to the polarity of the immediately adjacent electrodes to provide for omnidirectional and circumferential shrinkage of the vein. Thus, a relatively uniform RF field would be created along the circumference of the catheter by the alternating electrodes. In another embodiment, as shown in FIG. 24, if adjacent electrodes were to be moved closer together, two effective pairs of active electrodes of opposite polarity would be formed along the circumference of the catheter. While an RF field would still be formed along the entire circumference of the catheter, the RF field would be strongest between the closest adjacent electrodes of opposite polarity. Shrinkage of the vein would be concentrated where the RF field was strongest.

In another embodiment, the RF field may be further focused directionally using two pairs of electrodes arranged so as to be isolated from one another. For example, as shown in FIG. 25, the positive electrodes of each electrode pair would be adjacent to one another, and no field is formed along the circumference of the effective diameter between the two pairs of electrodes. Opposing RF fields are established by the two pairs of electrodes to create two discrete heating zones along the circumference. These heating zones may be directed to cause heating at isolated areas within the vein (i.e., not circumferentially) so as to direct treatment to the specific area of variceal bleeding from the vein. Specific or isolated instances of variceal bleeding may be treated by such directional application of RF energy to the vein.

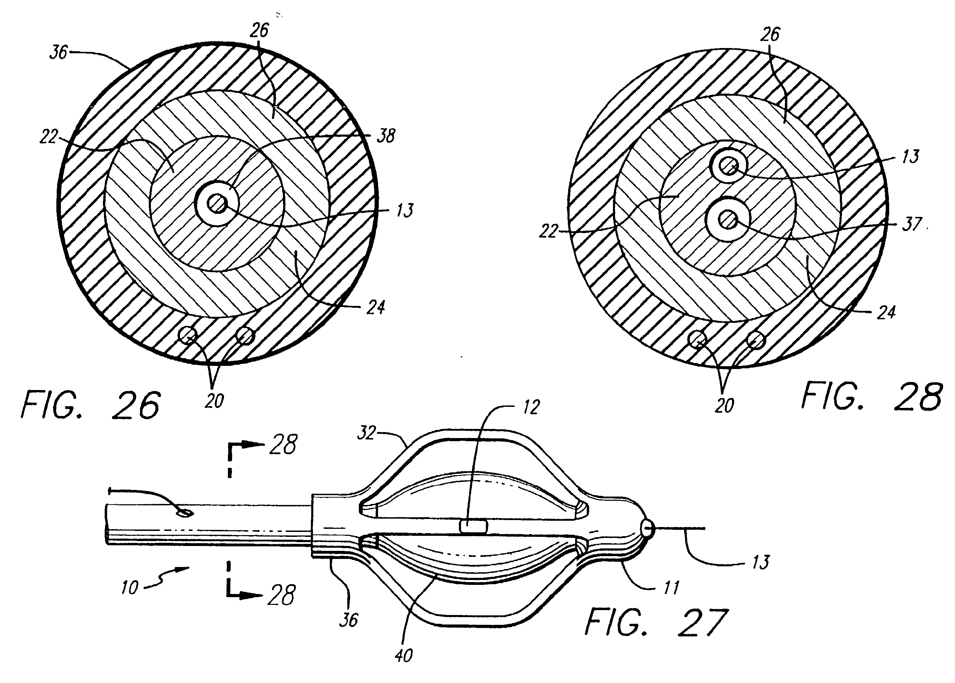

The working end of the catheter further includes a guide wire lumen 39 for accepting the guide wire 13. The tip of the guide wire 13 is preferably rounded. The guide wire lumen 39 is preferably insulated so as to prevent or minimize any coupling effect the electrodes 12 may have on the guide wire 13. The guide wire can be removed before the application of RF energy to the electrodes. A cross-sectional view of the catheter 10 taken along lines 26-26 of FIG. 20 is shown in FIG. 26. The guide wire 13 is shown centrally located within a guide wire lumen 38. The guide wire lumen 38 is surrounded by a layer of insulation material 22, which in turn is surrounded by a copper braid 24 for stability and stiffness, as well as for providing flexible torqueability to the catheter. An insulation sheath 26 covers the copper braid 24, and contains the conductive leads 20 to the electrodes as well. In a bipolar arrangement, the conductive leads 20 have opposing polarity. In an over-the-rail type catheter, the guide wire is outside the catheter until arriving at the working end of the catheter, upon which, the guide wire enters the guide wire lumen. The guide wire lumen 39 is preferably located within the insulation material 22 in order to electrically isolate the guide wire 13 from the electrodes 12. The guide wire lumen can also allow for the delivery or perfusion of medicant and cooling solution to the treatment area during application of the RF energy.

Another arrangement of the catheter 10, as shown in FIG. 27, includes bowable elongate members 32 having one end anchored to the working end 11 of the catheter, and the other end slidably connected to the catheter towards the connecting end. The catheter shown in FIG. 27 is similar to that shown in FIG. 20, except that instead of having the elongate members act as the electrodes themselves, the electrodes 12 are located on the elongate members 32. The elongate members 32 preferably include a flat central area for the electrodes 12. The central area remains substantially flat as the elongate members 32 are deflected and bowed outwardly. The substantially flat central area allows for a more uniform contact with the vein wall. The flat area establishes a larger surface area to assure contact between the electrode 12 on the elongate member and the vein wall. It is to be understood that the flat area need not be centrally located on the elongate member 32. The flat area should be located so as to be the first area that contacts the vein wall. The elongate members 32 at the working end of the catheter are connected to a movable tip manually controlled by a diameter actuator located at the connecting end of the catheter. The movable tip 17 is connected to the diameter actuator by an actuation wire 37 running centrally through the catheter, as shown in FIG. 28. The diameter actuator may be threaded onto the connecting end of the catheter. Maneuvering the diameter actuator into and out of the connecting end of the catheter causes a corresponding movement in the movable tip at the working end of the catheter via the actuation wire. If the movable tip 17 is pulled toward the connecting end by the diameter actuator, then the electrodes 12 are bowed outwardly. The bowed electrodes 12 preferably expand out to treat veins having diameters of up to ten mm or more. If the movable tip 17 is pushed forward by the actuator wire 37, the electrodes 12 are then retracted towards the shaft of the catheter. Contact between the electrode and the vein wall can be maintained with the vein wall as the vein shrinks.

In one embodiment, the balloon 40 is located between the catheter shaft and the elongate members 32. Manual manipulation of a sliding sleeve or a movable tip is not required in this embodiment, and the sliding sleeve, if used, need not travel any substantial length of the catheter. The balloon 40 may be either an elastic material, such as latex, or a noncompliant material. The balloon 40 is inflated and comes into contact with the elongate members 32. As the balloon 40 is further inflated, the electrodes 12 are moved outwardly in a radial direction as the elongate members are deflected and bowed by the expanding balloon 40. The balloon is preferably inflated using a non-conductive fluid, especially where the elongate members contain the electrodes, or where the elongate member itself is conductive so as to act as the electrode. When the proper diameter for the electrodes is reached, the inflation of the balloon ceases, and the application of the RF energy begins.

The balloon 40 covers a greater surface area over the venous treatment site, and ensures proper electrode placement relative to the vein wall while controlling the amount of venous shrinkage. More precise control over the shape and diameter of the balloon is possible using the bowable members. The balloon can also be used to control the effective diameter of the catheter at the working end. As RF energy is applied, the vein begins to shrink down to the effective diameter of the catheter. The effective diameter of the catheter is reduced under the control of the physician to control the amount of shrinkage. As the effective diameter is decreased, the electrodes continue to maintain apposition with the venous tissue. The application of RF energy from the electrodes 12 is terminated after shrinking the vein to the desired diameter, which is the final effective diameter as defined by the diameter of the balloon 40 and the deflected elongate members 32. The balloon 40 is then is deflated to a minimal profile. The elongate members 32 can be fabricated from stainless steel, spring steel, or nitinol so that the elongate members 32 would be biased to return to a reduced diameter profile when the balloon is deflated.

In another embodiment, the ends of the elongate members are instead slidably located within longitudinal slots or channels disposed along the circumference of the catheter. The ends of the bowable members would slide towards the working end within these channels as the members are deflected or bowed outwardly, and retract back towards the connecting end in order to return to their original configuration.

In another alternate arrangement single wire mesh or braided electrode, preferably when applying RF energy in a monopolar configuration. As before, the balloon could radially extend the mesh electrode outward into apposition with the vein wall. The balloon further controls the amount of vein shrinkage.

An alternative method for changing the effective diameter of the catheter is to move or deflect the electrodes into direct contact with the vein wall and then allow the vein wall to alter the effective diameter. As the electrodes emit RF energy, the vein wall shrinks and pushes the electrodes inwardly towards the catheter. The vein shrinkage reduces the effective diameter directly, rather than by the active control of the physician, thereby eliminating the need for constant fine mechanical adjustments to the effective diameter. A mechanism such as a push rod or fixed-diameter balloon may be included to prevent further radial contraction of the electrodes at a specific effective diameter, thereby controlling and limiting the amount of vein shrinkage. This has the advantage of maintaining the electrodes in apposition with the venous tissue so that the tissue is heated more than the surrounding blood, without requiring the physician to adjust the effective diameter of the catheter while applying the RF energy.

The method of using the present invention for the minimally invasive treatment of venous insufficiency can be performed using a catheter to deliver at least one electrode at the working end of the catheter to a venous treatment site in order to restore the proper function of a vein leading to the hemorrhoidal region. An over-the-wire or rail wire guided catheter can be used to deliver the one or more electrodes through the tortuous bends in the venous system to the hemorrhoidal treatment site.

The electrode applies RF energy at a suitable frequency to minimized coagulation for a sufficient amount of time to shrink, stiffen, and fixate the vein, yet maintain venous function or valvular competency. This intraluminal approach avoids the risks and morbidity associated with more invasive surgical techniques such as hemorrhoidectomy, while significantly reducing reflux of blood in the area without necrosing or removing the venous tissue.

When treating the veins of the lower hemorrhoidal region, the access site is prepped and a percutaneous introducer is inserted into the vein. The procedure for the repair of incompetent veins can be accomplished by a qualified physician with fluoroscopic guidance, ultrasonic observation, or direct visualization. A guide wire is passed into the vein through the introducer, and advanced through to the venous treatment site. Alternatively, the catheter may be inserted into the vein directly and manipulated without a guide wire. The guide wire preferably has a spring wound tip. The guide wire is advanced retrograde to the venous treatment site, such as the most distal incompetent vein site which is to be repaired. Several intravenous paths may be taken to the hemorrhoidal treatment site.

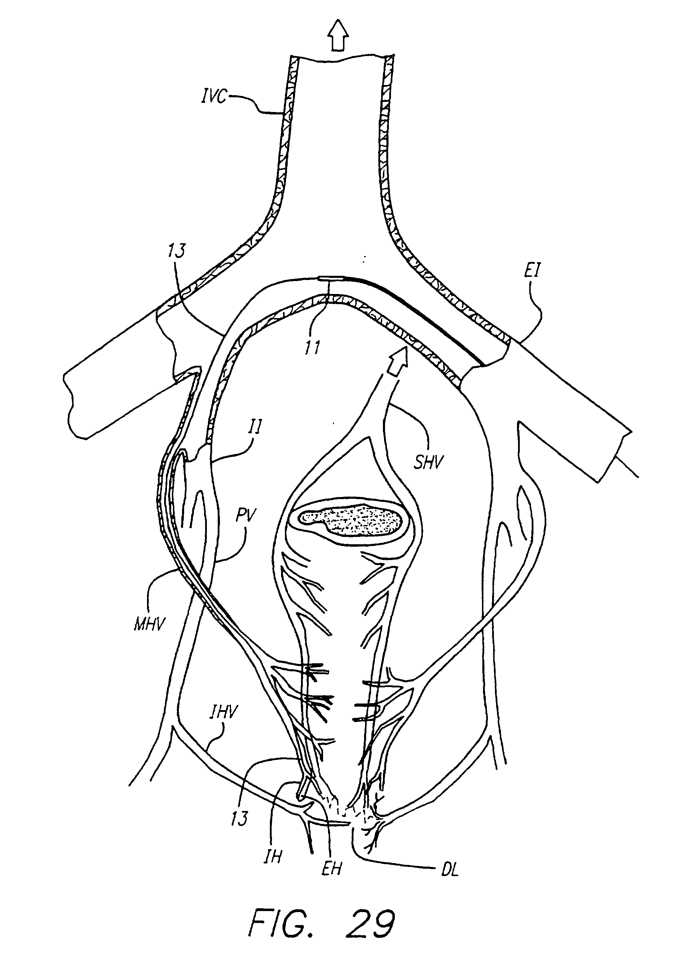

A partial cross-sectional view of the venous system leading to the hemorrhoidal region is shown in FIG. 29. Hemorrhoids are generally defined as internal or external depending on whether they are formed above or below the dentate line DL, respectively. Internal hemorrhoids IH are commonly formed when the smaller veins draining to the superior hemorrhoidal vein SHV or the middle hemorrhoidal vein MHV become dilated. External hemorrhoids are commonly formed when the smaller veins draining to the inferior hemorrhoidal vein IHV become dilated.

One method of delivering the catheter 10 and guide wire 13 is to introduce the guide wire 13 into the external iliac vein EI on the side opposite to the dilated veins of the hemorrhoid. The guide wire is steered across the bifurcated branch of the inferior vena cava IVC to the inferior iliac vein II. The guide wire is then maneuvered into either the middle hemorrhoidal vein MHV to treat internal hemorrhoids, or the pudendal vein PV and then the inferior hemorrhoidal vein IHV to treat external hemorrhoids. The guide wire 13 is deployed and maneuvered into the middle hemorrhoidal vein MHV to treat an internal hemorrhoid. The guide wire 13 is maneuvered through the venous system until it reaches the dilated veins of the hemorrhoid. The catheter 10 is then delivered to the venous treatment site over the guide wire 13, as shown in FIG. 29. The working end 11 of the catheter 10 includes one or more electrodes for applying RF energy once properly positioned at the venous treatment site to cause shrinkage of the vein. The working end of the catheter further includes a flexible nose cone tip to allow tracking of the catheter over the guide wire and through bends in the venous vascular system. Fluoroscopy, x-ray, ultrasound, or a similar imaging technique could be used to direct the specific placement of the catheter and to confirm position within the vein. X-ray contrast material can be injected through or around the catheter to identify the incompetent venous sections to be repaired. This approach advantageously allows the guide wire or catheter to avoid sharp bends or turns while being steered to the venous treatment site. It is to be understood that other access sites can be used to treat either internal or external hemorrhoids.

Another method of delivering the catheter and guide wire is to introduce the guide wire into the superior hemorrhoidal vein and maneuver the guide wire through the superior hemorrhoidal vein SHV to the hemorrhoidal region. The guide wire is maneuvered into position, and the catheter is then delivered over the guide wire to the venous treatment site for the internal hemorrhoid. The venous treatment site is within the lumen of a dilated vein.

When the electrodes 12 of the catheter 10 are positioned at the venous treatment site, an RF generator is activated to provide suitable RF energy, preferably at a low power level, and, preferably at a selected frequency from a range of 250 kHz to 350 MHZ. For example, one suitable frequency is 510 kHz. Another suitable frequency is 460 kHz. One criterion for the selection of the applied frequency is to control the spread, including the depth, of the thermal effect in the tissue. Another criteria for the selection of the applied frequency is the capability of filtering circuits to eliminate RF noise from thermocouple signals.

The energy emitted from the electrodes is converted within the venous tissue into heat. As the temperature of the venous tissue increases, the venous tissue begins to shrink. The shrinkage is due in part to dehydration and the structural transfiguration of the collagen fibers in the vein. Although the collagen becomes compacted during this process, the vessel wall collagen still retains elasticity.

RF energy can be applied to heat the dilated venous section of a hemorrhoid. The dilated vein is shrunk to a normal or reduced diameter under the controlled application of RF energy which heats the venous tissue. Venous pressure on the lower venous sections of the hemorrhoid may be lessened, due to the decrease in the cross-sectional area of the vein. Valve competency in the lower venous sections may also be restored indirectly by the lessening of the venous pressure. Thickening of the vein will also occur during treatment, which can reduce the likelihood of the recurrence of vein dilation. The temperature and power of the RF energy may also be controlled to both shrink the hemorrhoid and cause the wall of the hemorrhoidal vein to become affixed to adjacent tissue.

Although applying RF energy can shrink the vein dilation near the formation of the hemorrhoid, extending the shrinkage to include higher venous sections can be advantageous in further lessening the effect of higher and increased venous pressure on the hemorrhoidal system. A contiguous axial section of dilated vein can be treated by applying RF energy along the dilated venous section, even if the section is extensive. For example, hemorrhoids are sensitive to pressures from the portal system, which can be transferred to the hemorrhoids through the superior hemorrhoidal vein SHV. Treatment of the superior hemorrhoidal vein by general shrinkage along an extensive section of the vein above the hemorrhoid can offset the dilating forces that arise from any increased pressures from the portal system. Such treatment may be desirable even if there is no significant dilation in the superior hemorrhoidal vein SHV.

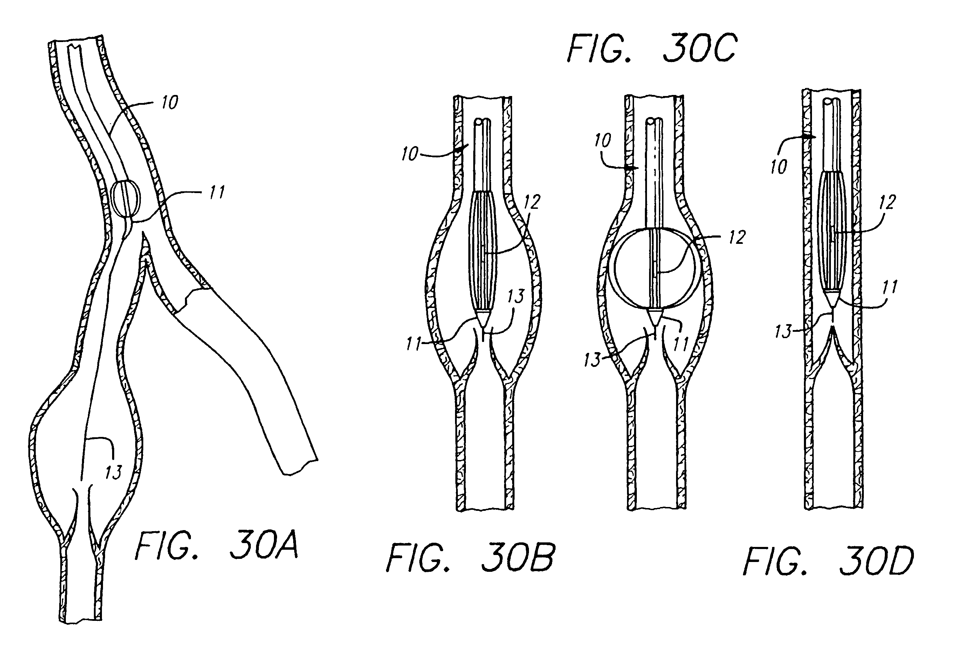

The catheter 10, as shown in Fig. 30a, is introduced over the guide wire 13 through the venous system. The tip 15 of the working end 11 of the catheter 10 is in the form of a nosecone which is flexible in order to travel over the guide wire and through bends in the venous system. As shown in FIG. 30b, the catheter 10 is delivered into the dilated venous section which may include an incompetent valve. The electrodes are then placed in apposition with the vein wall, preferably by mechanically bowing the electrodes 12 outwardly from the catheter 10 as shown in FIG. 30c. The application of RF energy from the electrodes causes the vein to shrink, and the effective diameter of the catheter, as defined by the bowed out electrodes, is mechanically decreased to control the amount of vein shrinkage. The bowed electrodes are held in position to define a specific effective diameter, as shown in FIG. 30d, to avoid occluding the vein. The catheter may be moved along the length of the dilated venous section to cause general shrinkage where the dilation is extensive.

RF energy is no longer applied from the electrodes after there has been sufficient shrinkage of the vein to alleviate the dilation of the vein. Substantial shrinkage may be achieved very rapidly, depending upon the specific treatment conditions, including the power level of the applied RF energy. The properties of the treatment site, such as temperature, can be monitored to provide feedback control for the RF energy. Other techniques such as impedance monitoring, and ultrasonic pulse echoing, can be utilized in an automated system which shuts down the application of RF energy from the electrodes to the venous section when sufficient shrinkage of the vein is detected and to avoid overheating or cauterization of the vein. Monitoring these values in an automatic feedback control system for the RF energy can also be used to control the power level and heating effect.

Sufficient shrinkage of the vein may be detected by fluoroscopy, venography, external ultrasound scanning, intravascular ultrasound scanning, impedance monitoring, temperature monitoring, direct visualization using an angioscope, or any other suitable method. For example, the catheter 10 can be configured to deliver an x-ray contrast medium to allow visualization by fluoroscopy for assessing the condition of the vein and the relationship of the catheter to the treatment area of the vein during the shrinkage process. As an alternative to fluoroscopy, external ultrasound techniques such as B-scanning using distinct ultrasound signals from different angles, or intravascular ultrasound can be used to acquire a more multidimensional view of the vein shrinkage at the treatment site, which improves the detection of uneven shrinkage in the vein. An angioscope may also be used to directly visualize and determine the extent and degree of vein shrinkage.

Where the catheter is designed with a fluid delivery lumen, a cooling fluid can be delivered through the delivery lumen to the bloodstream during RF heating of the vein being treated. The fluid may include radiodense contrast material. The delivered cooling fluid minimizes any heating effect on the blood, and reduces the risk of heating the blood to the point of coagulation. The fluid may be delivered through ports formed along the side of the catheter near the working end and the electrodes.

The working end 11 of the catheter 10 near the electrodes 12 can be used to physically limit the amount of shrinkage. The working end 11 is preferably sufficiently sized or enlarged to prevent the complete occlusion of the vein. Other schemes, such as an inflatable balloon, may be used to mechanically limit or control the amount of shrinkage in the vein or to displace blood from the treatment site. Such mechanical schemes can also be used to assure apposition between the electrodes and the venous tissue during treatment.

While providing for generalized shrinkage of the vein, the catheter may also be used to more directly treat the venous valves. The hemorrhoidal veins have bicuspid valves, and in a normal and competent valve, each cusp forms a sack or reservoir for blood which, under pressure, forces the surfaces of the cusps together to prevent retrograde flow of the blood and allow only antegrade flow to the heart. The arrows leading out the top of the inferior vena cava IVC and the superior hemorrhoidal vein SHV, as shown in FIG. 29, represent the antegrade flow of blood back to the heart. The venous valves prevent retrograde flow as blood is pushed forward through the vein lumen and back to the heart. In an incompetent valve, the cusps do not seal properly and retrograde flow of blood may occur. Incompetent valves may result from the stretching of dilated veins. As the valves fail, increased pressure is imposed on the lower veins and the lower valves of the vein, which in turn exacerbates the failure of these lower valves. Hemorrhoids may occur or become aggravated as a result. The valve cusps can experience some separation at the commissure due to the thinning and stretching of the vein wall at the cusps. When RF energy is applied within the dilated vein near the incompetent venous valve, shrinkage of the vein can restore valvular competency by reducing the dilation which is preventing the proper functioning of the venous valve.

In treating venous valves, the electrodes on the catheter are advanced until contact with the cusp of the venous valve is observed by fluoroscopy, ultrasound, or another detection method. The catheter is then pulled back slightly to allow treatment of the dilated section of vein. The electrodes are activated to deliver RF energy to the venous tissue and shrink the vein. The application of RF energy should be controlled to avoid unintentionally heating the valvular cusps. The shrinkage of the vein can be limited to prevent occlusion and allow the continued function of the vein. The outer diameter of the catheter or an extendable member can be controlled to limit the magnitude of the vein shrinkage.

After treatment, the commissure and the cusps of the venous valves should be closer together with little separation or prolapse, which indicates a restoration of the competency of the valve. Valvular competence may be determined by contrast injection or Doppler probe measurement. For example, a radiopaque contrast solution can be infused through the catheter lumen to assess valve competence via descending venography. It should be noted that reducing vein dilation by general shrinkage in a section above the section containing the incompetent venous valves could restore valvular competency by reducing the venous pressure on the valve and the dilation of the vein, which reduces the necessary span of the cusps. Also, direct placement of the electrodes across a vein valve can result in shrinkage of the loose, floppy leaflets, thereby preventing prolapse and reflux of blood through the valve.

The catheter 10 can be repositioned within the vein so as to treat as many venous sections and valves as necessary. RF energy is applied to each venous section to be repaired, until all of the desired venous sections are repaired and the valves are rendered competent. Multiple incompetent valves and dilated venous sections may be treated and repaired in a single minimally invasive procedure. If desired, a second introducer can be inserted into the patient in order to treat incompetent venous sections in the other vein systems, such as the superior hemorrhoidal vein.

Another area of venous insufficiency suitable for treatment in accordance with the present invention involves esophageal varices. Varicose veins called esophageal varices can form in the venous system along the submucosa of the lower esophagus, and bleeding can occur from the swollen veins. Properly sized catheters can be used in accordance with the present invention to deliver the electrodes to the site of venous insufficiency along the esophageal varices. Endovascular access for the catheter is preferably provided through the superior mesenteric vein or portal vein to shrink the portal vein branches leading to the lower esophagus. Proper positioning of the electrode within the vein can be confirmed using fluoroscopic or ultrasound techniques. The electrodes apply RF energy or other radiant energy at a suitable frequency to shrink the vein and reduce the swelling and transmission of high portal venous pressure to the veins surrounding the esophagus while maintaining the function of the vein. The amount of shrinkage of the vein can be limited by the diameter of the catheter or electrodes themselves can be expanded to a predetermined diameter which limits shrinkage of the vein to that diameter.

Varicose veins called esophageal varices can form in the venous system along the submucosa of the lower esophagus, and bleeding can occur from the swollen veins. A properly sized catheter 10 is used to deliver the electrodes 12 to the site of venous dysfunction along the esophageal varices. Endovascular access for the catheter is preferably provided through the superior mesenteric vein or portal vein to shrink the portal vein branches leading to the lower esophagus. Proper positioning of the electrode within the vein may be confirmed using fluoroscopic or ultrasound techniques. The electrodes apply RF energy or other forms of energy at a suitable power or frequency to shrink the vein and reduce the swelling and transmission of high portal venous pressure to the veins surrounding the esophagus while maintaining the function of the vein. The amount of shrinkage of the vein is limited by the diameter of the catheter itself, and the catheter or electrodes themselves may be expanded to a predetermined diameter which limits shrinkage of the vein to that diameter.

When treating the veins of the lower esophageal region, the access site is prepped and a percutaneous introducer is inserted into the vein. The procedure for the repair of incompetent veins may be accomplished by a qualified physician with fluoroscopic guidance or ultrasonic observation, or direct visualization. A guide wire 13 is passed into the vein through the introducer, and advanced through to the venous treatment site. The wire is advanced to the venous treatment site, such as the level of the most proximal incompetent vein site which is to be repaired. Preferably, the guide wire and catheter are advanced antegrade to the esophageal treatment site. Alternatively, the catheter may be inserted into the vein directly and manipulated without a guide wire.

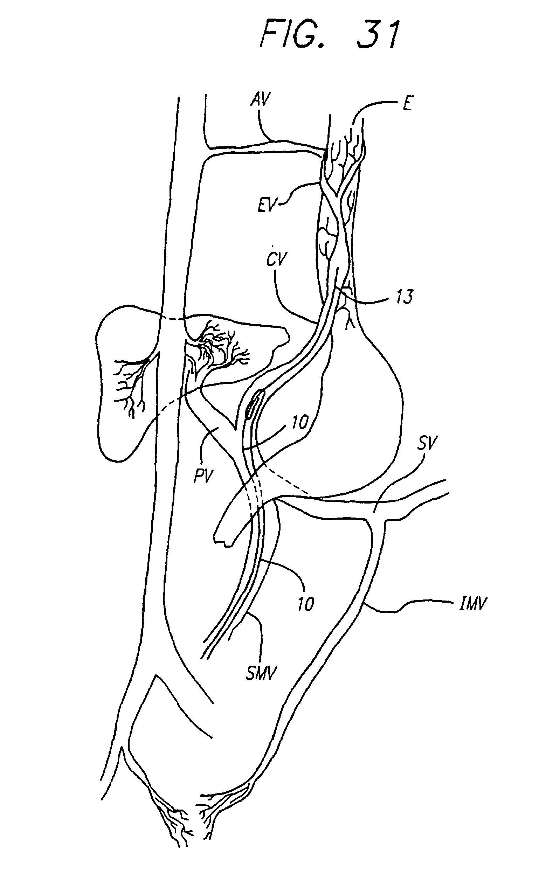

As shown in FIG. 31, in a partial view of the venous system leading to the esophageal region, the catheter 10 is advanced over the guide wire 13 to a dilated section of the vein. One method of delivering the catheter and guide wire is to introduce the guide wire through the superior mesenteric vein SMV to the portal vein PV and coronary vein CV which branches and leads to the lower esophagus E to form the esophageal veins EV. As an alternate route, the guide wire could be introduced into the inferior mesenteric vein, and routed through the splenic vein SV, the portal vein PV, and the coronary vein CV to arrive at the esophageal varix to be treated.

The guide wire is deployed and manipulated so as to reach the treatment site for treating the esophageal varices. The venous treatment site is preferably within the lumen of a dilated vein. The catheter 10 is then delivered to the venous treatment site over the guide wire 13 as shown in FIG. 31. Fluoroscopy, x-ray, ultrasound, or a similar imaging technique could be used to direct the specific placement of the catheter and to confirm position within the vein. X-ray contrast material may be injected through or around the catheter to identify the dilated venous sections to be treated. Hemorrhaging or bleeding of the esophageal varices may also be identified in this manner.

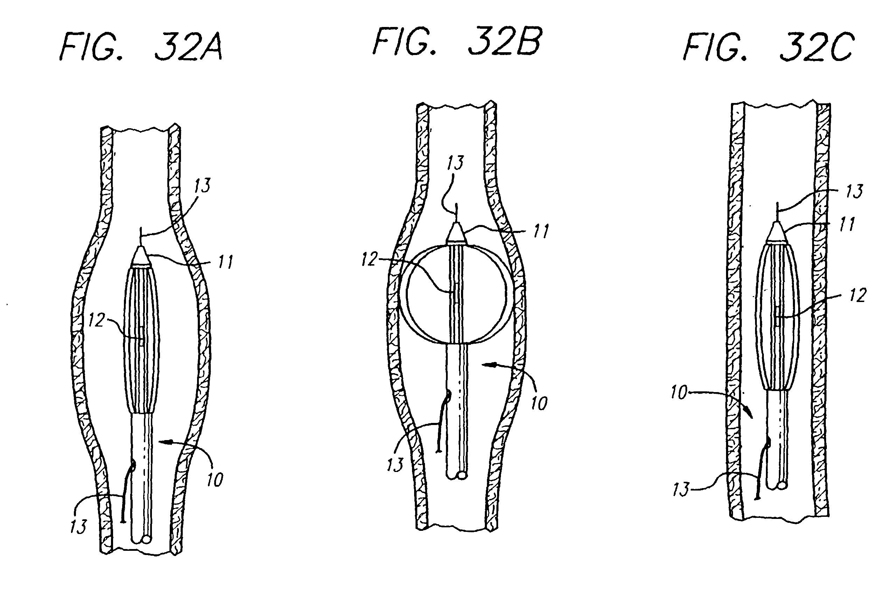

Once the dilated venous section is reached, the one or more electrodes 12 are activated to apply RF energy to the dilated venous section. While the electrodes may be maintained in the center of the vein, the electrodes are preferably placed in apposition with the vein wall. The electrodes in apposition with the venous tissue ensures that the heating effect is delivered towards the venous tissue, and not the blood moving through the vein, and allow control over the shrinkage of the vein. One method of achieving apposition is by bowing the electrodes out away from the body of the catheter. This is illustrated in FIGS. 32a, 32b, and 32c. The electrodes have an elongate longitudinal structure having opposite ends attached to a stationary and a moveable portion, respectively, at the working end of the catheter. The bowable electrodes are actuated by moving the outer sleeve of the catheter while maintaining the tip of the catheter stationary. Alternately a central wire could be used to move the tip while keeping the opposite end of the bowable electrode in place.

The one or more electrodes 12 at the working end 11 of the catheter 10 apply RF energy once properly positioned and apposed at the venous treatment site to cause shrinkage of the vein. An RF generator is activated to provide suitable RF energy to the electrodes, preferably at a low power level, and preferably at a selected frequency from a range of 250 kHz to 350 MHZ. For example, one suitable frequency is 510 kHz. One criteria for the selection of the applied frequency is to control the spread, including the depth, of the thermal effect in the tissue. Another criteria is compatibility with filter circuits which can be used to eliminate RF noise from thermocouple signals.

The energy emitted from the electrodes is converted within the venous tissue into heat. As the temperature of the venous tissue increases, the venous tissue begins to shrink. The shrinkage is due in part to dehydration and the structural transfiguration of the collagen fibers in the vein. Although the collagen becomes compacted during this process, the vessel with collagen still retains elasticity.

Substantial shrinkage may be achieved very rapidly, depending upon the specific treatment conditions, including the diameter of the vein being treated and power level of the applied RF energy. The properties of the treatment site, such as temperature, may be monitored to provide feedback control for the RF energy. Other techniques, such as impedance monitoring and ultrasonic pulse echoing, may also be utilized in an automated system which shuts down the application of RF energy from the electrodes to the venous section when sufficient shrinkage of the vein is detected and to avoid overheating or cauterization of the vein. Monitoring these values in an automatic feedback control system for the RF energy may also be used to control the heating effect.

Sufficient shrinkage of the vein may be detected by fluoroscopy, external ultrasound scanning, intravascular ultrasound scanning, impedance monitoring, temperature monitoring, direct visualization using an angioscope, or any other suitable method. For example, the catheter 10 may be configured to deliver x-ray contrast medium to allow visualization by fluoroscopy for assessing the condition of the vein and the relationship of the catheter to the treatment area of the vein during the shrinkage process. As an alternative to fluoroscopy, external ultrasound techniques such as B-scanning using distinct ultrasound signals from different angles, or intravascular ultrasound may be used to acquire a more multidimensional view of the vein shrinkage at the treatment site, which improves the detection of uneven shrinkage in the vein. An angioscope may also be used to directly visualize and determine the extent and degree of vein shrinkage.

The working end 11 of the catheter 10 near the electrodes 12 physically limits the amount of shrinkage. The electrodes 12 at the working end 11 are bowed outwards into apposition with the vein wall, and then gradually reduced inwardly towards the catheter during the application of RF energy. The final effective diameter of the electrodes 12 at the working end 11 is preferably sufficient to prevent the complete occlusion of the vein. Other schemes, such as an inflatable balloon, may be used to mechanically limit or control the amount of shrinkage in the vein to a desired diameter. RF energy is no longer applied from the electrodes after there has been sufficient shrinkage of the vein to alleviate the dilation of the vein. Methods other than the aforementioned mechanical methods may also be used to control the magnitude of vein shrinkage. Such non-mechanical methods include controlling the time and temperature of the venous RF treatment.

The dilated venous section is heated and shrunk to a normal or reduced diameter under the controlled application of RF energy in accordance with the present invention. A contiguous axial section of dilated vein may be treated by applying RF energy along the dilated venous section, even if the section is extensive. To treat an extensive venous section, the catheter is moved in intervals to progressively shrink the venous section, or moved back and forth along the extensive section during the application of RF energy. Further, thickening of the vein may occur during treatment, and reduce the likelihood of the recurrence of vein dilation and bleeding.

Applying RF energy shrinks the dilation of esophageal varices, and extending the shrinkage to include other sections in the portal venous system can be advantageous in further lessening the effect of increased venous pressure on the esophageal varices. Esophageal veins can be sensitive to pressures from the portal system. Treatment of the branches of the portal vein before the esophagus by general shrinkage along an extensive section of the vein before the esophagus can reduce the dilating effect on the esophageal veins caused by increased pressures from the portal venous system.

It is to be understood that other mechanisms may be employed to position or appose the electrodes with the venous section to be repaired without bowing or expanding the electrodes away from the catheter itself. The catheter may be made capable of being deflected, torqued, or otherwise moved to allow for the proper placement of the electrode. The catheter may be manufactured to provide a controllable bend near the working end. For example, the bend may be formed from a shape-memory metal, manipulatable by a system of wires, a torquable braid, or a permanent bend in the catheter. Manipulating the working end of the catheter enables preferential heating along the vein wall being treated, if desired, where the electrodes are placed closer to one side of the vein wall. Preferential heating of the vein can also be used to effect hemostasis.

It is to be understood that although a bipolar arrangement is described, a monopolar arrangement may also be used. In a monopolar arrangement, an inside electrode, such as a mesh or wire electrode, is inserted into a patient's body. An outer electrode having a much larger surface area than the inside electrode is placed on the outer surface of the patient's body near the treatment site. For example, an external metal plate is placed on the skin over the region to be treated by the inside electrode. Alternatively, a metalized balloon is introduced into the esophagus and inflated to come into contact with the mucosal lining of the esophagus to act as the inactive return electrode. The electrodes are connected to a RF generator which produces an electric field within the patient's body. Because the surface area of the inner electrode is much smaller than that of the outer electrode, the density of the electric field is much higher around the inside electrodes. The electric field reaches its highest density between the two electrodes in the region near the inside electrode. The increased density of the field around the inside electrode allows localized heating of the tissues surrounding the inside electrode. The degree of heating may be dependent on such factors as the impedance and dielectric constant of the tissue being heated. It is to be understood that different numbers and configurations of electrodes can be used to produce the desired discretionary heating effect.

As can be readily ascertained from the disclosure herein, the procedure of the present invention is accomplished without the need for prolonged hospitalization or postoperative recovery. The curative restoration of venous function is possible without the need for continued lifestyle changes, such as frequent leg elevation, the wearing of relatively uncomfortable elastic support stockings or prolonged treatment of recurrent venous stasis ulcers. Moreover, the need for surgical transplantation of veins would not be necessary.

Early treatment of venous disease could prevent more serious complications such as ulceration, thrombophlebitis and thromboembolism. The cost of treatment and complications due to venous disease would be significantly reduced. There would be no need for extensive hospitalization for this procedure, and the need for subsequent treatment and hospitalization would also be reduced from what is currently needed. Furthermore, the minimally invasive nature of the disclosed methods would allow the medical practitioner to repair or treat several venous sections in a single procedure in a relatively short period of time.

It is to be understood that the type and dimensions of the catheter and electrodes may be selected according to the size of the vein to be treated. Although the present invention has been described as treating venous insufficiency of the lower limb such as varicose veins in the leg, the present invention may be used to intraluminally treat venous insufficiency in other areas of the body.

Another area of venous insufficiency relates to erectile impotency of the penis. A significant number of all physically-induced cases of impotence result from excessive drainage of blood from the penile venous system. Venous-drainage-impotence can be treated using the present invention. Catheters having a sufficiently small diameter can be used to deliver the electrodes through the dorsal vein of the penile venous system to shrink this venous outflow path. Fluoroscopic or ultrasound techniques can be used to properly position the electrode within the incompetent vein. RF energy or other radiant energy is applied from the electrodes at a suitable frequency to shrink the surrounding venous tissue in order to reduce the excessive amount of drainage from the penis while maintaining venous function or valvular competency. The amount of shrinkage of the vein can be limited by the diameter of the catheter itself, or the catheter or electrodes themselves can be expanded to the appropriate size. Ligation of these veins should be avoided so as to allow for the proper drainage of blood from an engorged penis which is necessary for proper penile function.

While several particular forms of the invention have been illustrated and described, it will be apparent that various modifications can be made without departing from the scope of the invention. Accordingly, it is not intended that the invention be limited, except as by the appended claims.

## Claims

1. An apparatus for applying energy to reduce the diameter of a vein, the apparatus comprising;
a catheter (10) having a working end and an outer diameter, the outer diameter of the catheter being adapted to be less than an inner diameter of the vein, the catheter (10) further including a moveable outer sleeve (36);
a heating device (12) adapted to heat a venous treatment area to reduce the diameter of the vein, the heating device being located at the working end of the catheter (10); and
at least one bowable member (32) being adapted to move the heating device (12) outwards from the catheter (10), the bowable member (32) being conductive and the bowable member further including an insulating film (35) covering the surface of the conductive bowable member (32) except for an uncovered portion that contacts the vein wall when the bowable member is moved outwardly from the catheter (15), the bowable member (32) including a first end and a second end, the first end being connected to the working end of the catheter, the second end being connected to the moveable outer sleeve (36) so that the uncovered portion of the bowable member can be moved into apposition with the vein by manipulating the outer sleeve, wherein the heating device (12) includes the uncovered portion of the conductive bowable member, and the bowable member having sufficient structural strength to prevent a reduction in the diameter of the vein beyond an effective diameter defined by the bowable member, the effective diameter being greater than the outer diameter of the catheter.

2. The apparatus of claim 1, further **characterized in that** the conductive bowable member (32) has a rectangular shape with a flat section between the first end and the second end, wherein the flat section contains the uninsulated portion.

3. The apparatus of any of the preceding claims, further comprising a balloon (40) located between the bowable member (12) and the catheter (10), wherein the balloon engages and forces the bowable member radially outward when the balloon is inflated.

4. The apparatus of any of the preceding claims, wherein the heating device (12) comprises at least one electrode.

5. The apparatus of claim 4, further **characterized in that** the at least one electrode further includes a plurality of longitudinal electrodes arranged along the circumference of the working end (11) of the catheter so as to provide omnidirectional heating along a section of the catheter (10).

6. The apparatus of claim 5, further **characterized in that** each of the longitudinal electrodes has a polarity opposite to the polarity of immediately adjacent longitudinal electrodes.

7. The apparatus of claim 5, further **characterized in that** there are an even number of longitudinal electrodes arranged on the catheter (10) so as to provide effective pairs of longitudinal electrodes.

8. The apparatus of claim 5, further **characterized in that** there are an even number of electrodes arranged circumferentially on the catheter (10) so as to provide effective pairs of electrodes, wherein each of the electrodes has a polarity opposite to the polarity of the immediately adjacent electrodes.

9. The apparatus of claim 5, further **characterized in that** there are an even number of electrodes arranged circumferentially on the catheter (10) so as to provide effective pairs of electrodes, wherein a first electrode has a first polarity, a second electrode adjacent to the first electrode has a second polarity opposite to the first polarity, and a third electrode adjacent to the first electrode has the same polarity as the first polarity, wherein there are an even number of electrodes arranged on the catheter so as to provide effective pairs of electrodes to apply energy in a directional manner.

10. The apparatus of any of the preceding claims, further comprising a positioning device (40) to bring the heating device (12) into apposition with the venous treatment area of the vein.

11. The apparatus of any of the preceding claims, further comprising an occluding balloon (40) located at the working end of the catheter, the occluding balloon being configured to occlude the vein when inflated.

12. The apparatus of any of the preceding claims, further comprising a sensor (60) located at the working end of the catheter (10), and a microprocessor adapted to receive a signal from the sensor, said signal representing a condition at the treatment area, the microprocessor comprising means for controlling the heating device (12) such that the spread of the heating effect in the venous tissue in response to the signal from the sensor is controlled.

13. The apparatus of any of the preceding claims, further comprising a microprocessor comprising means for selecting a frequency for the heating device (12) such that the spread of the heating effect in the venous tissue is controlled.

14. The apparatus of any of the preceding claims, further **characterized in that** the catheter further includes a lumen adapted for injecting fluid into the vein.

15. The-apparatus of any claims 5-14, further **characterized in that** the catheter further includes a guide wire contained in an insulated guide wire lumen, said guide wire lumen adapted to prevent electrical coupling between the guide wire and electrode.

## Patentansprüche

1. Vorrichtung zur Anwendung von Energie, um den Durchmesser einer Vene zu reduzieren, wobei die Vorrichtung umfasst:
einen Katheter (10) mit einem Funktionsende und einem äußeren Durchmesser, wobei der äußere Durchmesser des Katheters so eingestellt werden kann, dass er kleiner als der innere Durchmesser der Vene ist, wobei der Katheter (10) weiterhin eine bewegliche äußere Ummantelung (36) aufweist;
eine Heizvorrichtung (12) zum Erhitzen eines Behandlungsbereichs in der Vene, um den Durchmesser der Vene zu vermindern, wobei die Heizvorrichtung am Funktionsende des Katheters (10) angeordnet ist; und
mindestens ein biegsames Element (32) zum Bewegen der Heizvorrichtung (12) entlang des Katheters (10) nach außen, wobei das biegsame Element (32) leitfähig ist und weiterhin einen isolierenden Film (35) aufweist, welcher die Oberfläche des leitfähigen biegsamen Elements (32) bedeckt, mit Ausnahme eines nicht bedeckten Teils, welcher die Wand der Vene kontaktiert, wenn das biegsame Element entlang des Katheters (15) nach außen bewegt wird, wobei das biegsame Element (32) ein erstes Ende und ein zweites Ende enthält, wobei das erste Ende mit dem Funktionsende des Katheters und das zweite Ende mit der beweglichen äußeren Ummantelung (36) verbunden ist, so dass der nicht bedeckte Teil des biegsamen Elements durch Manipulation der äußeren Ummantelung in der Vene positioniert werden kann, wobei die Heizvorrichtung (12) den unbedeckten Teil des leitfähigen biegsamen Elements einschließt, und das biegsame Element eine ausreichende strukturelle Festigkeit aufweist, um eine Verminderung des Durchmessers der Vene unter einen effektiven Durchmesser, welcher durch das biegsame Element bestimmt wird, zu verhindern, wobei der effektive Durchmesser größer als der äußere Durchmesser des Katheters ist.

2. Vorrichtung nach Anspruch 1, weiterhin **dadurch gekennzeichnet, dass** das leitfähige biegsame Element (32) eine rechteckige Form mit einem flachen Abschnitt zwischen dem ersten Ende und dem zweiten Ende aufweist, wobei der flache Abschnitt den nicht isolierten Teil enthält.

3. Vorrichtung nach einem der vorhergehenden Ansprüche, weiterhin enthaltend einen Ballon (40), der zwischen dem biegsamen Element (12) und dem Katheter (10) angeordnet ist, wobei der Ballon beim Aufblasen auf das biegsame Element einwirkt und es radial nach außen drückt.

4. Vorrichtung nach einem der vorhergehenden Ansprüche, wobei die Heizvorrichtung (12) mindestens eine Elektrode umfasst.

5. Vorrichtung nach Anspruch 4, weiterhin **dadurch gekennzeichnet, dass** die mindestens eine Elektrode weiterhin eine Vielzahl von longitudinalen Elektroden umfasst, welche entlang des Umfangs des Funktionsendes (11) des Katheters angeordnet sind, um ein in alle Richtungen wirkendes Erhitzen entlang eines Abschnitts des Katheters (10) zu bewirken.

6. Vorrichtung nach Anspruch 5, weiterhin **dadurch gekennzeichnet, dass** jede longitudinale Elektrode eine gegensätzliche Polarität zu den unmittelbar angrenzenden longitudinalen Elektroden hat.

7. Vorrichtung nach Anspruch 5, weiterhin **dadurch gekennzeichnet, dass** auf dem Katheter (10) eine gerade Anzahl von longitudinalen Elektroden angeordnet ist, so dass zusammen wirkende Paare von longitudinalen Elektroden bereitgestellt werden.

8. Vorrichtung nach Anspruch 5, weiterhin **dadurch gekennzeichnet, dass** auf dem Katheter (10) eine gerade Anzahl von longitudinalen Elektroden angeordnet ist, so dass zusammen wirkende Paare von longitudinalen Elektroden bereitgestellt werden, wobei jede Elektrode eine gegensätzliche Polarität zu den unmittelbar angrenzenden Elektroden hat.

9. Vorrichtung nach Anspruch 5, weiterhin **dadurch gekennzeichnet, dass** auf dem Umfang des Katheters (10) eine gerade Anzahl an Elektroden angeordnet ist, so dass zusammen wirkende Paare von Elektroden bereitgestellt werden, wobei eine erste Elektrode eine erste Polarität hat, eine zweite Elektrode, die angrenzend an die erste Elektrode angeordnet ist, eine zweite Polarität hat, die der ersten Polarität gegensätzlich ist, und eine dritte Elektrode, die angrenzend an die erste Elektrode angeordnet ist, die gleiche Polarität wie die erste Polarität hat, wobei eine gerade Anzahl von Elektroden auf dem Katheter angeordnet ist, so dass zusammen wirkende Paare von Elektroden bereitgestellt werden, um Energie in gerichteter Form einzubringen.

10. Vorrichtung nach einem der vorhergehenden Ansprüche, weiterhin umfassend eine Positioniervorrichtung (40), um die Heizvorrichtung (12) mit der Behandlungsfläche in der Vene zu positionieren.

11. Vorrichtung nach einem der vorhergehenden Ansprüche, weiterhin umfassend einen an dem Funktionsende des Katheters angeordneten Okklusionsballon (40), der beim Aufblasen eine Okklusion der Vene bewirkt.

12. Vorrichtung nach einem der vorhergehenden Ansprüche, weiterhin umfassend einen an dem Funktionsende des Katheters (10) angeordneten Sensor (60), und einen Mikroprozessor, der ein Signal des Sensors empfangen kann, wobei das Signal einen Zustand an der Behandlungsfläche wiedergibt, wobei der Mikroprozessor eine Einrichtung zum Steuern der Heizvorrichtung (12) umfasst, so dass die Ausbreitung des Heizeffekts in dem venösen Gewebe als Reaktion auf das Signal von dem Sensor gesteuert wird.

13. Vorrichtung nach einem der vorhergehenden Ansprüche, weiterhin umfassend einen Mikroprozessor mit einer Einrichtung zur Auswahl der Frequenz der Heizvorrichtung (12), so dass die Ausbreitung des Heizeffekts in dem venösen Gewebe gesteuert wird.

14. Vorrichtung nach einem der vorhergehenden Ansprüche, weiterhin **dadurch gekennzeichnet, dass** der Katheter ein Lumen enthält, durch welches Flüssigkeit in die Vene injiziert werden kann.

15. Vorrichtung nach einem der Ansprüche 5 bis 14, weiterhin **dadurch gekennzeichnet, dass** der Katheter einen Führungsdraht enthält, welcher sich in einem isolierten Lumen für den Führungsdraht befindet, wobei das Lumen für den Führungsdraht so angepasst ist, dass eine elektrische Kopplung zwischen dem Führungsdraht und der Elektrode verhindert wird.

## Revendications

1. Appareil pour appliquer de l'énergie pour réduire le diamètre d'une veine, l'appareil comprenant :
un cathéter (10) ayant une extrémité de travail et un diamètre externe, le diamètre externe du cathéter étant conçu pour être plus petit qu'un diamètre interne de la veine, le cathéter (10) comprenant en outre un manchon externe mobile (36) ;
un dispositif de chauffage (12) apte à chauffer une zone de traitement veineuse pour réduire le diamètre de la veine, le dispositif de chauffage se situant à l'extrémité de travail du cathéter (10) ; et
au moins un élément (32) apte à se courber, apte à faire sortir le dispositif chauffage (12) du cathéter (10), l'élément apte à se courber (32) étant conducteur, et l'élément apte à se courber comprenant en outre un film isolant (35) couvrant la surface de l'élément conducteur apte à se courber (32) à l'exception d'une partie non-couverte qui vient en contact avec la paroi de la veine lorsque l'élément apte à se courber est amené à sortir du cathéter (15), l'élément apte à se courber (32) comprenant une première extrémité et une seconde extrémité, la première extrémité étant reliée à l'extrémité de travail du cathéter, la seconde extrémité étant reliée au manchon externe mobile (36) de sorte que la partie non-couverte de l'élément apte à se courber peut être amenée en apposition avec la veine en manipulant le manchon externe, où le dispositif de chauffage (12) comprend la partie non-couverte de l'élément conducteur apte à se courber, et l'élément apte à se courber ayant une résistance structurelle suffisante pour éviter une réduction du diamètre de la veine au-delà d'un diamètre effectif défini par l'élément apte à se courber, le diamètre effectif étant plus grand que le diamètre externe du cathéter.

2. Appareil selon la revendication 1, **caractérisé en outre en ce que** l'élément conducteur apte à se courber (32) possède une forme rectangulaire avec une section plate entre la première extrémité et la seconde extrémité, où la section plate contient la partie non-isolée.

3. Appareil selon l'une des revendications précédentes, comprenant en outre un ballonnet (40) situé entre l'élément apte à se courber (12) et le cathéter (10), où le ballonnet vient en prise avec et force l'élément apte à se courber radialement vers l'extérieur lorsque le ballonnet est gonflé.

4. Appareil selon l'une des revendications précédentes, où le dispositif de chauffage (12) comprend au moins une électrode.

5. Appareil selon la revendication 4, **caractérisé en outre en ce qu**'au moins une électrode comprend en outre plusieurs électrodes longitudinales agencées le long de la circonférence de l'extrémité de travail (11) du cathéter de manière à produire un chauffage omnidirectionnel le long d'une section du cathéter (10).

6. Appareil selon la revendication 5, **caractérisé en outre en ce que** chacune des électrodes longitudinales possède une polarité opposée à la polarité des électrodes longitudinales directement adjacentes.

7. Appareil selon la revendication 5, **caractérisé en outre en ce qu**'il y a un nombre égal d'électrodes longitudinales agencées sur le cathéter (10) de manière à former des paires effectives d'électrodes longitudinales.

8. Appareil selon la revendication 5, **caractérisé en outre en ce qu**'il y a un nombre égal d'électrodes agencées circonférentiellement sur le cathéter (10) de manière à former des paires effectives d'électrodes, où chacune des électrodes possède une polarité opposée à la polarité des électrodes directement adjacentes.

9. Appareil selon la revendication 5, **caractérisé en outre en ce qu**'il y a un nombre égal d'électrodes agencées circonférentiellement sur le cathéter (10) de manière à former des paires effectives d'électrodes, où une première électrode possède une première polarité, une deuxième électrode adjacente à la première électrode possède une deuxième polarité opposée à la première polarité, et une troisième électrode adjacente à la première électrode possède la même polarité que la première polarité, où il y a un nombre égal d'électrodes agencées sur le cathéter de manière à former des paires effectives d'électrodes pour appliquer l'énergie d'une manière directionnelle.

10. Appareil selon l'une des revendications précédentes, comprenant en outre un dispositif de positionnement (40) pour amener le dispositif de chauffage (12) en apposition avec la zone de traitement veineuse de la veine.

11. Appareil selon l'une des revendications précédentes, comprenant en outre un ballonnet d'occlusion (40) situé à l'extrémité de travail du cathéter, le ballonnet d'occlusion étant configuré pour occlure la veine lorsqu'elle est gonflée.

12. Appareil selon l'une des revendications précédentes, comprenant en outre un capteur (60) situé à l'extrémité de travail du cathéter (10), et un microprocesseur apte à recevoir un signal du capteur, ledit signal représentant un état à la zone de traitement, le microprocesseur comprenant un moyen pour commander le dispositif de chauffage (12) de façon que la propagation de l'effet de chauffage dans le tissu veineux en réponse au signal du capteur est contrôlée.

13. Appareil selon l'une des revendications précédentes, comprenant en outre un microprocesseur comprenant un moyen pour sélectionner une fréquence pour le dispositif de chauffage (12) de telle sorte que la propagation de l'effet de chauffage dans le tissu veineux est contrôlée.

14. Appareil selon l'une des revendications précédentes, **caractérisé en outre en ce que** le cathéter comprend en outre une lumière apte à injecter un fluide dans la veine.

15. Appareil selon l'une des revendications 5 à 14, **caractérisé en outre en ce que** le cathéter comprend en outre un fil de guidage se trouvant dans une lumière de fil de guidage isolée, ladite lumière de fil de guidage étant apte à éviter un couplage électrique entre le fil de guidage et l'électrode.
